# EUROPEAN PATENT SPECIFICATION

(11) **EP 1 053 020 B1**
(45) Date of publication and mention of the grant of the patent: **31.03.2004**
(21) Application number: 99903216.2
(22) Date of filing: 20.01.1999
(51) Int. Cl.: A61K 47/48

(54) **ABSORBABLE MICROPARTICLES**
ASORBIERBARE MIKROPARTIKEL
MICROPARTICULES ABSORBABLES

(30) Priority: 29.01.1998 US 15394
(43) Date of publication of application: 22.11.2000
(73) Proprietor: Poly-Med Inc., Anderson, SC 29625 (US)
(72) Inventor: SHALABY, Shalaby, Wahba, Anderson, SC 29625 (US)
(74) Representative: Lunt, Mark George Francis
(86) International application number: PCT/US1999/001180
(87) International publication number: WO 1999/038536

(56) References cited:
- EP-A- 0 626 170
- WO-A-92/11844
- WO-A-95/03356
- DATABASE CHEMABS CHEMICAL ABSTRACTS SERVICE, COLUMBUS, OHIO, US DN=129:235478, CORBETT, J. ET AL: "Injectable, absorbable gel-formers for controlled release of antibiotics" XP002106940 & PROC. INT. SYMP. CONTROLLED RELEASE BIOACT. MATER. (1998), 25TH, 38-39 CODEN: PCRMEY;ISSN: 1022-0178,

## Description

This invention pertains to a sustained release complex of one or more peptide, one or more protein or a combination thereof ionically immobilized on an absorbable polymer microparticle optionally having an absorbable polymer coating. The microparticle complex of this invention comprises a peptide(s) and/or protein(s) which have at least one amino group and/or at least one carboxyl group per molecule and a solid absorbable polyester microparticle having surface and subsurface carboxylic groups or amino groups in sufficient amounts to bind the peptide(s) and/or protein(s) so that the immobilized peptide(s) or protein(s) represent 0.1% to 30% of the total mass of the microparticle complex. The microparticle complex with immobilized peptide(s) and/or protein(s) are optionally further encased individually or in groups with an absorbable polymer to control, further, the release of the immobilized peptide(s) and/or protein(s). To control the release of the immobilized peptide(s) and/or protein(s) even further, the encased microparticles can be incorporated into a composition with an absorbable gel-forming liquid that transforms to a flexible gel or semisolid upon contacting water in the biologic environment.

Many drug delivery systems have been developed, tested and utilized for the controlled *in vivo* release of pharmaceutical compositions. For example, polyesters such as poly(DL-lactic acid), poly(glycolic acid), poly(ε-caprolactone) and various other copolymers have been used to release biologically active molecules such as progesterone; these have been in the form of microcapsules, films or rods (M. Chasin and R. Langer, editors, Biodegradable Polymers as Drug Delivery Systems, Dekker, NY 1990). Upon implantation of the polymer/therapeutic agent composition, for example, subcutaneously or intramuscularly, the therapeutic agent is released over a specific period of time. Such bio-compatible biodegradable polymeric systems are designed to permit the entrapped therapeutic agent co diffuse from the polymer matrix. Upon release of the therapeutic agent, the poller is degraded in vivo, obviating surgical removal of the implant. Although the factors that contribute to poller degradation are not well understood, it is believed that such degradation for polyesters may be regulated by the accessibility of ester linkages to non-enzymatic autocatalytic hydrolysis of the polymeric components.

Several EPO publications and U.S. Patents have addressed issues of polymer matrix design and its role in regulating the rate and extent of release of therapeutic agents *in vivo*.

For example, Deluca (EPO Publication 0 467 389 A2) describes a physical interaction between a hydrophobic biodegradable polymer and a protein or polypeptide. The composition formed was a mixture of a therapeutic agent and a hydrophobic polymer that sustained its diffusional release from the matrix after introduction into a subject.

Hutchinson (U.S. Pat. No. 4,767,628) controlled the release of a therapeutic agent by uniform dispersion in a polymeric device. It is disclosed that this formulation provides for controlled continuous release by the overlap of two phases: first, a diffusion-dependent leaching of the drug from the surface of the formulation; and second, releasing by aqueous channels induced by degradation of the polymer.

Other in-situ forming biodegradable implants and methods of forming them are described in U.S. Pat. Nos. 5,278,201 ('201 Patent) and U.S. Pat. No. 5,077,049 ('049 Patent), to Dunn et al. The Dunn et al. patents disclose methods for assisting the restoration of periodontal tissue in a periodontal pocket and for retarding a migration of epithelial cells along the root surface of a tooth. The '049 Patent discloses methods which involve placement of an in-situ forming biodegradable barrier adjacent to the surface of the tooth. The barrier is microporous and includes pores of defined size and can include biologically active agents. The barrier formation is achieved by placing a liquid solution of a biodegradable polymer, such as poly(dl-lactide-co-glycolide) water-coagulatable, thermoplastic in a water miscible, non-toxic organic solvent such as N-methyl pyrrolidone (i.e., to achieve a typical polymer concentration of about 50%) into the periodontal pocket. The organic solvent dissipates into the periodontal fluids and the biodegradable, water coagulatable polymer forms an in-situ solid biodegradable implant. The dissipation of solvent creates pores within the solid biodegradable implant to promote cell ingrowth. The '859 Patent likewise discloses methods for the same indications involving the formation of the biodegradable barrier from a liquid mixture of a biodegradable, curable thermosetting prepolymer, curing agent and water-soluble material such as salt, sugar, and water-soluble polymer. The curable thermosetting prepolymer is described as an acrylic-ester terminated absorbable polymer.

In addition, a number of systems for the controlled delivery of biologically active compounds to a variety of sites are disclosed in the literature. For example, U.S. Patent No. 5,011,692, to Fujioka et al., discloses a sustained pulsewise release pharmaceutical preparation which comprises drug-containing polymeric material layers. The polymeric material layers contain the drug only in a slight amount, or free of the drug. The entire surface extends in a direction perpendicular to the layer plane and is coated with a polymeric material which is insoluble in water. These types of pulsewise-release pharmaceutical dosages are suitable for embedding beneath the skin.

U.S. Pat. No. 5,366,756, to Chesterfield et al., describes a method of preparing porous bioabsorbable surgical implant materials. The method comprises providing a quantity of particles of bioabsorbable implant material, and coating particles of bioabsorbable implant material with at least one growth factor. The implant can also contain antimicrobial agents.

U.S. Patent No. 5,385,738, to Yamhira et al., discloses a sustained-release injection system, comprising a suspension of a powder comprised of an active ingredient and a pharmaceutically acceptable biodegradable carrier (e.g., proteins, polysaccharides, and synthetic high molecular weight compounds, preferably collagen, atelo collagen, gelatin, and a mixture thereof) in a viscous solvent (e.g., vegetable oils, polyethylene glycol, propylene glycol, silicone oil, and medium-chain fatty acid triglycerides) for injection. The active ingredient in the pharmaceutical formulation is incorporated into the biodegradable carrier in the following state: (i) the active ingredient is chemically bound to the carrier matrix; (ii) the active ingredient is bound to the carrier matrix by intermolecular action; or (iii) the active ingredient is physically embraced within the carrier matrix.

Moreover , such systems as those previously described in the literature, for example, such as by Dunn, et al. (U.S. Pat. No. 4,938,763), teach in-situ formations of biodegradable, microporous, solid implants in a living body through coagulation of a solution of a polymer in an organic solvent such as N-methyl-2-pyrrolidine. However, the use of solvents, including those of low molecular organic ones, facilitates migration of the solution from the application site thereby causing damage to living tissue including cell dehydration and necrosis. Loss of the solvent mass can lead to shrinkage of the coagulum and separation from surrounding tissue.

US Patent No. 5,612,052 describes cation-exchanging microparticles made typically of carboxyl-bearing polyester chains onto which basic bioactive agents are immobilized to provide a control release system within an absorbable gel-forming liquid polyester. The contents of US Patent 5,612,052 is incorporated herein by reference. Conjugating carboxylic entities, ionically, with basic polypeptide has been noted in the prior art as described in US Patent No. 5,672,659 and US Patent No. 5,665,702. However, these complexes are soluble chemical entities formed by molecularly reacting the individual basic and carboxylic components in their respective solutions to form a well-defined ion-conjugate as a new chemical entity with physicochemical properties. This is distinguished from the present invention where the complex formation takes place in a heterogeneous system involving primarily surface complex formation.

### SUMMARY OF THE INVENTION

The present invention is directed to a bound microparticle comprising an absorbable heterochain polymer core and one or more peptide, one or more protein or a combination thereof ionically immobilized on said absorbable heterochain polymer core,
wherein each peptide is independently selected from the group consisting of growth hormone releasing peptide (GHRP), luteinizing hormone-releasing hormone (LHRH), somatostatin, bombesin, gastrin releasing peptide (GRP), calcitonin, bradykinin, galanin, melanocyte stimulating hormone (MSH), growth hormone releasing factor (GRF), amylin, tachykinins, secretin, parathyroid hormone (PTH), enkaphelin, endothelin, calcitonin gene releasing peptide (CGRP), neuromedins, parathyroid hormone related protein (PTHrP), glucagon, neurotensin, adrenocorticothrophic hormone (ACTH), peptide YY (PYY), glucagon releasing peptide (GLP), vasoactive intestinal peptide (VIP), pituitary adenylate cyclase activating peptide (PACAP), motilin, substance P, neuropeptide Y (NPY), TSH and analogs and fragments thereof or a pharmaceutically acceptable salt thereof; and
wherein each protein is independently selected from the group consisting of growth hormone, erythropoietin, granulocyte-colony stimulating factor, granulocyte-macrophage-colony stimulating factor and interferons.

A preferred bound microparticle of the immediately foregoing, denoted group B, is where said peptide, protein or a combination thereof or a pharmaceutically acceptable salt thereof comprises 0.1% to 30% of the total mass of the bound microparticle.

A preferred bound microparticle of the immediately foregoing, denoted group C, is where said absorbable heterochain polymer core comprises glycolate units.

A preferred bound microparticle of the immediately foregoing, denoted group D, is where the absorbable heterochain polymer core further comprises citrate residues, tartrate residues or malate residues.

A preferred bound microparticle of the immediately foregoing, denoted group E, is where the ratio of glycolate units to citrate residues, to tartrate residues or to malate residues is about 7-1 to about 20-1.

Another preferred bound microparticle of group C is where said glycolate units terminate with a carboxyl moiety.

Yet another preferred bound microparticle of group C is where said glycolate units terminate with an amine moiety.

In another aspect, this invention provides an encased microparticle comprising one or more of a bound microparticle within an absorbable encasing polymer
wherein said bound microparticle comprises an absorbable heterochain polymer core and one or more peptide, one or more protein or a combination thereof immobilized on said absorbable heterochain polymer core,
where each peptide is independently selected from the group consisting of growth hormone releasing peptide (GHRP), luteinizing hormone-releasing hormone (LHRH), somatostatin, bombesin, gastrin releasing peptide (GRP), calcitonin, bradykinin, galanin, melanocyte stimulating hormone (MSH), growth hormone releasing factor (GRF), amylin, tachykinins, secretin, parathyroid hormone (PTH), enkaphelin, endothelin, calcitonin gene releasing peptide (CGRP), neuromedins, parathyroid hormone related protein (PTHrP), glucagon, neurotensin, adrenocorticothrophic hormone (ACTH), peptide YY (PYY), glucagon releasing peptide (GLP), vasoactive intestinal peptide (VIP), pituitary adenylate cyclase activating peptide (PACAP), motilin, substance P, neuropeptide Y (NPY), TSH and analogs and fragments thereof or a pharmaceutically acceptable salt thereof;
each protein is independently selected from the group consisting of growth hormone, erythropoietin, granulocyte-colony stimulating factor, granulocyte-macrophage-colony stimulating factor and interferons; and
where said absorbable heterochain polymer core comprises glycolate units.

A preferred encased microparticle of the immediately foregoing is where said peptide, protein or combination thereof or pharmaceutically acceptable salt thereof comprises 0.1% to 30% of the total mass of the bound microparticle, and where said absorbable heterochain polymer core further comprises citrate residues, tartrate residues or malate residues.

A preferred encased microparticle of the immediately foregoing, denoted group F, is where the ratio of glycolate units to citrate residues, to tartrate residues or to malate residues is about 7-1 to about 20-1 and said glycolate units terminate with a carboxyl moiety or an amine moiety.

A preferred encased microparticle of the immediately foregoing is where said absorbable encasing polymer comprises
(a) l-lactide based units and glycolide based units,
(b) d,l-lactide based units and glycolide based units,
(c) d,l-lactide based units or
(d) l-lactide based units and d,l-lactide based units.

A preferred encased microparticle of the immediately foregoing is where the ratio of l-lactide based units to glycolide based units is about 75-25 to about 90-10, the ratio of 1-lactide based units to d,l-lactide based units is about 80-20 and the ratio of d,l-lactide based units to glycolide based units is about 75-25 to about 90-10.

A preferred encased microparticle of group F is where the absorbable encasing polymer constitutes 5 to 70% of the total mass of the encased microparticle.

A preferred encased microparticle of the immediately foregoing is where the absorbable encasing polymer constitutes 20-60% of the total mass of the encased microparticle.

A preferred encased microparticle of the immediately foregoing is where the absorbable encasing polymer constitutes 30-50% of the total mass of the encased microparticle.

In another aspect, this invention provides a pharmaceutical composition comprising the bound microparticles described above and a pharmaceutically acceptable carrier.

In another aspect, this invention provides a pharmaceutical composition comprising the bound microparticles described above, a non-aqueous absorbable gel-forming liquid polyester and optionally a pharmaceutically acceptable carrier.

In another aspect, this invention provides a pharmaceutical composition comprising the encased microparticles described above and a pharmaceutically acceptable carrier.

In another aspect, this invention provides a pharmaceutical composition comprising the encased microparticles described above, a non-aqueous absorbable gel-forming liquid polyester and optionally a pharmaceutically acceptable carrier.

Another preferred bound microparticle of group D, denoted group G, is where the absorbable heterochain polymer core comprises citrate residues and the peptide is an LHRH analog.

A preferred bound microparticle of the immediately foregoing is where the ratio of glycolate units to citrate residues of the absorbable heterochain polymer core is about 7-1 to about 20-1 and where the LHRH analog is p-Glu-His-Trp-Ser-Tyr-D-Trp-Leu-Arg-Pro-Gly-NH₂.

Another preferred bound microparticle of group D, denoted group H, is where the absorbable heterochain polymer core comprises tartrate residues and the peptide is an LHRH analog.

A preferred bound microparticle of the immediately foregoing is where the ratio of glycolate units to tartrate residues is about 7-1 to about 20-1 and the LHRH analog is p-Glu-His-Trp-Ser-Tyr-D-Trp-Leu-Arg-Pro-Gly-NH₂.

Yet another preferred bound microparticle of group D, denoted group I, is where the absorbable heterochain polymer core comprises citrate residues and the peptide is a somatostatin analog.

A preferred bound microparticle of the immediately foregoing is where the ratio of glycolate units to citrate residues is about 7-1 to about 20-1 and the somatostatin analog is H-β-D-Nal-Cys-Tyr-D-Trp-Lys-Val-Cys-Thr-NH₂ where the two Cys are bonded by a disulfide bond, N-hydroxyethylpiperazinyl-acetyl-D-Phe-Cys-Tyr-D-Trp-Lys-Abu-Cys-Thr-NH₂ where the two Cys are bonded by a disulfide bond or N-hydroxyethylpiperazinyl-ethylsulfonyl-Phe-Cys-Tyr-D-Trp-Lys-Abu-Cys-Thr-NH₂ where the two Cys are bonded by a disulfide bond.

Yet another preferred bound microparticle of group D, denoted group J, is where the absorbable heterochain polymer core comprises tartrate residues and the peptide is a somatostatin analog.

A preferred bound microparticle of the immediately foregoing is where the ratio of glycolate units to tartrate residues is about 7-1 to about 20-1 and the somatostatin analog is H-β-D-Nal-Cys-Tyr-D-Trp-Lys-Val-Cys-Thr-NH₂, where the two Cys are bonded by a disulfide bond, N-hydroxyethylpiperazinyl-acetyl-D-Phe-Cys-Tyr-D-Trp-Lys-Abu-Cys-Thr-NH₂ where the two Cys are bonded by a disulfide bond or N-hydroxyethylpiperazinyl-ethylsulfonyl-Phe-Cys-Tyr-D-Trp-Lys-Abu-Cys-Thr-NH₂ where the two Cys are bonded by a disulfide bond.

A preferred encased microparticle of this invention is an encased microparticle comprising one or more bound microparticles of group G encased within an absorbable encasing polymer which comprises
(a) l-lactide based units and glycolide based units,
(b) d,l-lactide based units and glycolide based units,
(c) d,l-lactide based units or
(d) l-lactide based units and d,l-lactide based units.

A preferred encased microparticle of the immediately foregoing is where the ratio of glycolate units to citrate residues of the absorbable polymer core is about 7-1 to about 20-1, the LHRH analog is p-Glu-His-Trp-Ser-Tyr-D-Trp-Leu-Arg-Pro-Gly-NH₂ and where the ratio of:
(a) l-lactide based units to glycolide based units is about 75-25 to about 90-10,
(b) d,l-lactide based units to glycolide based units is about 75-25 to about 90-10 and
(c) l-lactide based units to d,l-lactide based units is about 80-20.

Another preferred encased microparticle comprises one or more bound microparticles of group H encased within an absorbable encasing polymer which comprises
(a) l-lactide based units and glycolide based units,
(b) d,l-lactide based units and glycolide based units,
(c) d,l-lactide based units or
(d) l-lactide based units and d,l-lactide based units.

A preferred encased microparticle of the immediately foregoing is where the ratio of glycolate units to tartrate residues of the absorbable polymer core is about 7-1 to about 20-1, the LHRH analog is p-Glu-His-Trp-Ser-Tyr-D-Trp-Leu-Arg-Pro-Gly-NH₂ and where the ratio of:
(a) 1-lactide based units to glycolide based units is about 75-25 to about 90-10,
(b) d,l-lactide based units to glycolide based units is about 75-25 to about 90-10 and
(c) l-lactide based units to d,l-lactide based units is about 80-20.

Another preferred encased microparticle comprises one or more bound microparticles of group I encased within an absorbable encasing polymer which comprises
(a) l-lactide based units and glycolide based units,
(b) d,l-lactide based units and glycolide based units,
(c) d,l-lactide based units or
(d) l-lactide based units and d,l-lactide based units.

A preferred encased microparticle of the immediately foregoing is where the ratio of glycolate units to citrate residues of the absorbable polymer core is about 7-1 to about 20-1, the somatostatin analog is H-β-D-Nal-Cys-Tyr-D-Trp-Lys-Val-Cys-Thr-NH₂ where the two Cys are bonded by a disulfide bond, N-hydroxyethylpiperazinyl-acetyl-D-Phe-Cys-Tyr-D-Trp-Lys-Abu-Cys-Thr-NH₂ where the two Cys are bonded by a disulfide bond or N-hydroxyethylpiperazinyl-ethylsulfonyl-Phe-Cys-Tyr-D-Trp-Lys-Abu-Cys-Thr-NH₂ where the two Cys are bonded by a disulfide bond;
and where the ratio of:
(a) l-lactide based units to glycolide based units is about 75-25 to about 90-10,
(b) d,l-lactide based units to glycolide based units is about 75-25 to about 90-10 and
(c) l-lactide based units to d,l-lactide based units is about 80-20.

Another preferred encased microparticle comprises one or more bound microparticles of group J and an absorbable encasing polymer which comprises
(a) l-lactide based units and glycolide based units,
(b) d,l-lactide based units and glycolide based units,
(c) d,l-lactide based units or
(d) l-lactide based units and d,l-lactide based units.

A preferred encased microparticle of the immediately foregoing is where the ratio of glycolate units to tartrate residues of the absorbable polymer core is about 7-1 to about 20-1, the somatostatin analog is H-β-D-Nal-Cys-Tyr-D-Trp-Lys-Val-Cys-Thr-NH₂ where the two Cys are bonded by a disulfide bond, N-hydroxyethylpiperazinyl-acetyl-D-Phe-Cys-Tyr-D-Trp-Lys-Abu-Cys-Thr-NH₂ where the two Cys are bonded by a disulfide bond or N-hydroxyethylpiperazinyl-ethylsulfonyl-Phe-Cys-Tyr-D-Trp-Lys-Abu-Cys-Thr-NH₂ where the two Cys are bonded by a disulfide bond;
and where the ratio of:
(a) l-lactide based units to glycolide based units is about 75-25 to about 90-10,
(b) d,l-lactide based units to glycolide based units is about 75-25 to about 90-10 and
(c) l-lactide based units to d,l-lactide based units is about 80-20.

In another aspect, this invention provides a process for making an encased microparticle as described above comprising the step of encasing a bound microparticle with an absorbable encasing polymer.

A preferred process of the immediately foregoing is where a dispersion of said bound microparticles in a solution comprising said absorbable encasing polymer and a solvent is dropped onto a pre-cooled medium, where said medium is not a solvent of said absorbable encasing polymer.

A preferred process of the immediately foregoing is where the solution of the absorbable encasing polymer consists of about 5% to 30% of the absorbable encasing polymer, the pre-cooled medium is an alcohol having two or more carbon atoms and the temperature of the medium is room temperature to about -80°C.

A preferred process of the immediately foregoing is where the temperature of the pre-cooled medium is about -60°C to -80°C and the medium is isopropyl alcohol.

In yet another aspect, this invention provides a process for making an encased microparticle as described above comprising the step of encasing a bound microparticle with an absorbable encasing polymer using an emulsion technique.

### DETAILED DESCRIPTION

The term "absorbable" as used herein, means a water insoluble material such as a polymer which undergoes chain disassociation in the biological environment to water soluble by-products.

The term "microparticle" as used herein, refers to the particles of absorbable polyester, which are preferably in essentially spherical form.

The term "bound microparticle" as used herein, refers to a microparticle having one or more peptide and/or one or more protein ionically immobilized on the microparticle.

The term "encased microparticle" as used herein, refers to a bound microparticle having a polymer coating, where the polymer coating is not necessarily completely occlusive.

The term "polymer core" as used herein, is another way of referring to microparticles.

The term "encasing polymer" as used herein, refers to the polymer that is used to encase a bound microparticle.

The term "gel-forming liquid polyester" as used herein, refers to materials which absorb solvents such as water, undergo phase transformation and maintain three dimensional networks capable of reversible deformation.

The instant application denotes amino acids using the standard three letter abbreviation known in the art, for example Ala = alanine.

A microparticle of the present invention is crystalline and is made of an absorbable polyester, such as polyglycolide having one or more carboxylic groups on the individual chains which results in a sufficient concentration of carboxylic groups on the surface of the microparticle and immediate subsurface of the microparticle to complex and ionically immobilize a peptide(s) and/or a protein(s) having one or more basic groups. Or the carboxylic groups of the polyglycolide can be amidated, for example by a diamine, preferably a primary or secondary amine or a mixture thereof, wherein the amine forms a complex that ionically immobilizes a peptide(s) and/or a protein(s) having one or more acidic groups. Since the surface of the microparticles is not necessarily homogeneous, the term "subsurface" refers to the crevices and the like found on the surface of the microparticles. The bound microparticles provide a means for the controlled release of a peptide(s) and/or protein(s) in a patient. To further control the release of the immobilized peptide(s) and/or protein(s), the bound microparticles can be encased individually or in groups with an absorbable polymer coating. The bound microparticles release the peptide(s) and/or protein(s) over a period of about two days to about three months in a patient, preferably about one week to about three months. The encased microparticles release the peptide(s) and/or protein(s) over a period of about three days to six months in a patient, preferably about two weeks to five months.

Typical examples of a peptide that can be immobilized on a microparticle include but are not limited to growth hormone releasing peptide (GHRP), luteinizing hormone-releasing hormone (LHRH), somatostatin, bombesin, gastrin releasing peptide (GRP), calcitonin, bradykinin, galanin, melanocyte stimulating hormone (MSH), growth hormone releasing factor (GRF), amylin, tachykinins, secretin, parathyroid hormone (PTH), enkaphelin, endothelin, calcitonin gene releasing peptide (CGRP), neuromedins, parathyroid hormone related protein (PTHrP), glucagon, neurotensin, adrenocorticothrophic hormone (ACTH), peptide YY (PYY), glucagon releasing peptide (GLP), vasoactive intestinal peptide (VIP), pituitary adenylate cyclase activating peptide (PACAP), motilin, substance P, neuropeptide Y (NPY), TSH, and analogs and fragments thereof. Examples of proteins that can be immobilized on a microparticle are growth hormone, erythropoietin, granulocyte-colony stimulating factor, granulocyte-macrophage-colony stimulating factor and interferons.

A microparticle can be made of a lactide based polymer or a solid semi-crystalline polylactone such as polyglycolide which can be formed by ring opening polymerization of acid-bearing hydroxylic initiators such as glycolic, lactic, malic, tartaric, and citric acid. A microparticle of the present invention can be synthesized according to the following procedure. In a reaction vessel are mixed a lactide based monomer and/or a lactone such as glycolide and an acid initiator such as tartaric acid, malic acid or citric acid. The reaction vessel is warmed to about 35-45°C, preferably 40°C and put under vacuum for about 20-60 minutes, preferably 30 minutes. The temperature of the reaction vessel is raised to about 105-115°C, preferably 110°C. Once this temperature is reached the vessel is placed under an atmosphere of oxygen-free nitrogen, and the mixture is stirred. Once the mixture melts, a catalytic amount of an organometallic catalyst suitable for ring opening polymerization, such as stannous 2-ethyl-hexanoate solution in a non-protic solvent, such as toluene is added. A vacuum Is reapplied for about 30-90 seconds to remove toluene without significant removal of monomer. The temperature of the mixture is raised to about 115-125°C, preferably 120°C for about 5-10 minutes before further raising it to about 145-150°C. It was kept at this temperature for about 3-5 hours, preferably 4 hours, under constant mechanical stirring.

The resulting polymer is micronized by initially grinding it using a Knife-grinder. The polymer is then micronized in an Aljet Micronizer using a pressurized dry nitrogen stream. The mean particle diameter size is analyzed in a Malvern Mastersizer/E using a volume distribution model and 200/5 cS silicone oil as dispersant.

The polymer is purified and the sodium salt thereof is formed by dispersing the micronized polymer in acetone and placing it in a sonicator, preferably for about 30 minutes. During this time the dispersion was also homogenized at about 8,000-24,000 rpm, preferably 9,500 rpm, using a homogenizer. After this sonication/ homogenization step the dispersion is centrifuged at about 3,000-7,000 rpm, preferably 5,000 rpm preferably for about 30 minutes in a centrifuge. The supernatant is discarded, the centrifuge cakes re-suspended in fresh acetone, and the sonication/homogenization step repeated. Once the second centrifugation is complete, the supernatant is discarded and the cakes were re-suspended in deionized water. One final sonication/homogenization step is then carried out to remove any remaining acetone and the dispersion is once again centrifuged at about 5,000 rpm for about 30 minutes.

The centrifuge cakes are re-suspended in fresh deionized water and the pH of the dispersion is monitored. Sufficient volumes of a weak base such as 0.2M sodium carbonate solution are added with stirring to raise the pH to between about pH 8 and about pH 9. The dispersions are allowed to stir for about 30 minutes before being vacuum-filtered over filter paper. The filter cakes are rinsed with further deionized water, frozen, and lyophilized.

Purification is monitored by differential scanning calorimetry (DSC) with a heating rate of about 5°C/min to 15°C/min, preferably 10°C/min.

An anion-exchanger microparticle is obtained by taking the cation-exchanger microparticles and incubating it in hot dilute solution (∼80°C) of a diamine, it is preferred that the amines can be both a primary amine or both a secondary amine or a mixture of a primary and a secondary amine, of known concentration in dioxane or THF under an inert gas such as argon. The concentration of the diamine in dioxane or THF is determined by acidimetry. When the reaction practically ceases to take place, the amidated microparticles are separated by filtration, rinsed with dioxane or THF, and dried under reduced pressure.

A peptide(s) and/or protein(s) can be immobilized on a microparticle according to the following method. The sodium salt of a microparticle is dispersed in solutions containing the free-base of a peptide(s) and/or protein(s) dissolved in water. The dispersions are incubated at room temperature with stirring for about 2 hours before filtering out the bound microparticles. The filter cakes are rinsed with further deionized water, frozen, and lyophilized. Samples are then analyzed for nitrogen by elemental analysis to determine the amount of the peptide(s) and/or protein(s) immobilized.

The size of a microparticle plays a role in the amount of a peptide and/or protein that a microparticle of the instant invention can immobilize. The smaller the size of a microparticle, the more surface area a mass of microparticles possess and, thus, the more peptide and/or protein can be immobilized per mass of microparticles. Size reduction of the microparticles to micron or sub-micron dimensions can be achieved as described above. The diameter of the microparticles can range in size from about 0.5 µm to 100 µm, preferably 1µm to 15 µm and more preferably 3 µm to 10 µm.

The absorbable encasing polymer can be a crystalline or non-crystalline lactide/glycolide copolymer, amorphous l-lactide/d,l-lactide co-polymer, caprolactone/ glycolide copolymer or trimethylene carbonate/glycolide copolymer, that is soluble in conventional organic solvents, such as chloroform, methylene chloride, acetone, acetonitrile, ethyl acetate, and ethyl formate. Non-solvents of such an absorbable encasing polymer include water, low boiling temperature alcohols and hydrocarbons. The absorbable encasing polymers can be synthesized by catalyzing ring-opening polymerization of lactones, or by polymerization of cyclic monomers such as ∈-caprolactone, p-dioxanone, trimethylene carbonate, 1,5-dioxepan-2-one or 1,4-dioxepan-2-one in the presence of a chain initiator, such as a hydroxy polycarboxylic acid. Still another method involves reacting an organic polycarboxylic acid with a pre-formed polyester, which is disclosed in U.S. Patent No. 5,612,052, the contents of which is incorporated herein by reference.

The encasing of the bound microparticles can be achieved by phase separation of an emulsion. An alternate encasing method entails the use of an ultrasonic atomizer where a dispersion of the bound microparticles in an absorbable encasing polymer solution is introduced as micro-droplets into a cooled non-solvent medium. Bound microparticles are encased with an absorbable encasing copolymer of lactide and glycolide using traditional microencapsulation or coating techniques of solid particles such as the emulsion evaporation method described by H. Demian and S.W. Shalaby for encapsulating barium sulfate microparticles as disclosed in U.S. Patent application USSN: 08/467,361, the contents of which are incorporated herein by reference, or by coagulation of solid microparticles encased in a polymer solution and delivered through an ultrasonic atomizer (nebulizer) into a liquid medium that is a non-solvent for the encasing polymer, but where the liquid medium non-solvent is capable of extracting the solvent of the encasing polymer solution about the encased solid microparticles. Depending on the concentration of the polymer solution for encasing the microparticles, the number of the original bound microparticles in the encased microparticles can vary from 1 to several hundred with an average diameter of an encased microparticle ranging from 0.5 µm to 100 µm.

The following method relates to the preparation of encased peptide-loaded and/or protein-loaded (hereinafter peptide-loaded) cation exchangers by nebulization. The encasing copolymer of interest is dissolved in a solvent, such as either acetonitrile, ethyl acetate or ethyl formate at a concentration of between 10 and 30% (W/W). A sufficient weight of this solution is used for dispersion of the peptide-loaded CE so that the weight ratio of peptide-loaded CE to encasing copolymer ranges from about 30:70 to about 80:20. Dispersion is achieved by high speed homogenization. The dispersion is fed at a flow rate of between 1ml/min and 10 ml/min to an ultrasonic atomization nozzle with variable frequency - this frequency can be altered from 12kHz to 35kHz - higher frequency allows higher flow rates while maintaining particle characteristics. The dispersion is thus nebulized into a collecting sink made up of at least 1 to 10 times excess of isopropanol or ethanol (compared to the volume of encasing copolymer solvent used) containing sufficient dry-ice pellets (usually 0.5 - 1Kg by weight per liter of IPA) so that the temperature of the slurry remains between -70° and -80°C throughout the nebulization. This slurry is stirred at between 300 and 700 rpm depending on its volume. In the case of acetonitrile as solvent, the nebulization droplets will freeze immediately on contact with the slurry. Once nebulization is complete the entire dispersion is allowed to thaw of its own accord to between 10°C and room temperature before vacuum filtering. The filter cakes are rinsed with de-ionized water to remove excess non-solvent. The particles obtained have the appearance of smooth microspheres in the case of a predominantly d,l-lactide encasing copolymer; they appear slightly wrinkled when the encasing copolymer is mainly l-lactide based.

The binding capacity of a microparticle ion-exchanger can be determined as follows. For example, for a cation-exchanger microparticle, available carboxylic groups, in a predetermined mass of the microparticles, are neutralized using cold dilute aqueous sodium carbonate solution of known normality. The neutralized microparticles are isolated by filtration and rinsed thoroughly with cold deionized water and then air dried. The solid microparticles are then incubated in dilute solution of Pilocarpine hydrochloride of known concentration so as to provide a slight excess of the basic drug over that predicted from the binding capacity data. The concentration of the remaining Pilocarpine HCl in the aqueous medium is monitored for a period of time until no significant change in the base pick-up by the microparticles can be recorded. The percent of immobilized base on the microparticles is determined from the exhaustion data and then verified by elemental analysis for nitrogen.

The binding capacity of the anion-exchanger (amidated particles) is determined by (1) elemental analysis for nitrogen and (2) extent of binding to Naproxen by measuring the extent of Naproxen removed from a dilute solution using HPLC. The latter is confirmed by release of the immobilized Naproxen with a dilute sodium hydroxide solution of known concentration.

The bound microparticles or the encased microparticles of this invention can be administered to a patient via administration routes well known to those of ordinary skill in the art, such as parenteral administration, oral administration or topical administration. Preferably, it is administered as a powder or a suspension via intranasal route or as an inhalant through the pulmonary system. When it is administered parenterally it is preferable that it is administered as a dispersion in an isotonic aqueous medium or in a non-aqueous, absorbable gel-forming liquid polyester as described in U.S. Patent No. 5,612,052, the contents of which are incorporated herein by reference. The formulations comprising bound microparticles and/or encased microparticles of the present invention can also include a variety of optional components. Such components include, but are not limited to, surfactants, viscosity controlling agents, medicinal agents, cell growth modulators, dyes, complexing agents, antioxidants, other polymers such as carboxymethyl cellulose, gums such as guar gum, waxes/oils such as castor oil, glycerol, dibutyl phthalate and di(2-ethylhexyl)phthalate as well as many others. If used, such optional components comprise form about 0.1% to about 20%, preferably from about 0.5% to about 5% of the total formulation.

The effective dosages of bound microparticles or encased microparticles to be administered to a patient can be determined by the attending physician or veterinarian and will be dependent upon the proper dosages contemplated for the peptide(s) and/or protein(s) and the quantity of the peptide(s) and/or protein(s) immobilized on the microparticles. Such dosages will either be known or can be determined by one of ordinary skill in the art.

The preparation of gel-formers is disclosed in US Patent No. 5,612,052, the contents of which is incorporated herein by reference. Specific examples of gel formers are described below.

### Preparation of 80/20 (by weight) Block Copolymers of 60/40 Trimethylene

Carbonate/Glycolide and Polyethylene Glycol-400 (GF-1): A flame-dried resin kettle equipped with a mechanical stirrer and a nitrogen inlet was charged with polyethylene glycol-400 (0.299 mole, 119.5 g), stannous octoate (0.2 M in toluene, 4.700 ml, 0.946 mmole), glycolide (1.78 mole, 206.5 g) and trimethylene carbonate (2.65 mole, 270 g). The reactor was purged with argon several times and then heated to melt and then heated to and stirred at about 150°C for about 12 hours. At the conclusion of the reaction, the temperature was lowered while maintaining fluidity and excess monomer was removed under reduced pressure. The resulting polymer was analyzed by infrared and NMR for composition and gel-permeation chromatography for molecular weight.

### Preparation of 15/85 (by weight) Block Copolymer of 60/40 Trimethylene

Carbonate/Glycolide and Polyethylene Glycol-400 (GF-2): The title copolymer was synthesized according to the procedure described for GF-1 but using polyethylene glycol-400 (1.063 mole, 425 g), stannous octoate (0.2 M in toluene, 1,760ml, 0.35 mmole), glycolide (0.279 mole, 32.4 g) and trimethylene carbonate (0.418 mole, 42.6 g) and stirring for about 9 hours.

### Preparation of 80/20 (by weight) Block Copolymer of 90/10 Trimethylene

Carbonate/Glycolide and Polyethylene Glycol-1500 (GF-3): The title copolymer was synthesized according to the procedure described for GF-1 but using polyethylene glycol-1500 (0.267 mole, 400 g), stannous octoate (0.2 M in toluene, 1200 ml, 0.247 mmole), glycolide (0.097 mole, 11.2 g) and trimethylene carbonate (0.87 mole, 88.7 g) and stirring for about 13 hours.

### Example I

### Preparation, Micronization, and Purification of Poly(glycolic acid) polymers initiated with Citric Acid (PGCA) for use as Cation Exchangers (CE)

**Example I(a): 7/1 PGCA-** A 500 ml glass reactor was loaded with 242.63 g of glycolide (Purac Biochem, Arkelsedijk, The Netherlands) and 57.37 g of citric acid (Aldrich, Gillingham, Dorset, U.K.). The citric acid had been further dried over silica gel (Fisher Scientific, Loughborough, Leics., U.K.) in an Abderhalden apparatus (Aldrich, St. Louis, Missouri, USA). The reactor was immersed in an oil bath at about 40°C and put under vacuum (0.04 mbar) for about 30 minutes. The bath was then lowered and it's temperature raised to about 110°C. Once this temperature was reached the reactor was placed under an atmosphere of oxygen-free nitrogen and re-immersed. The contents were stirred at about 100 rpm using a Heidolph stirrer (Heidolph Elektro GmbH, Kelheim, Germany). Once the reactor contents melted 1.09 ml of a 0.1M stannous 2-ethyl-hexanoate solution (Sigma, St. Louis, Missouri, USA) in toluene (Riedel de-Haen, Seelze, Germany) was added (stoichiometric ratio of 50 ppm). A vacuum was reapplied via a liquid nitrogen trap for about 30 seconds to remove toluene without significant removal of monomer. The oil bath temperature was then raised to about 120°C for about 5 minutes before further raising it to about 150°C. It was kept at this temperature for about 4 hours under constant mechanical stirring of about 100 rpm. The title polymer was obtained.
**Example I(b): 10/1 PGCA-** The title polymer was obtained by following the procedure of Example Ia, but using 257.40 g of glycolide, 42.60 g of citric acid and 1.10 ml of a 0.1M stannous 2-ethyl-hexanoate solution in toluene (stoichiometric ratio of 50 ppm).
**Example I(c): 15/1 PGCA- 15/1 PGCA-** A flame-dried resin kettle equipped with a mechanical stirrer and an argon inlet was charged with glycolide (2.586 mole, 300 g), anhydrous citric acid (0.172 mole, 33 g), and stannous octoate (0.2 M in toluene, 862ml, 0.172 mmole). The polymerization reactor and its contents were purged with dry argon several times. After melting the polymerization charge, the reactants were heated and stirred at about 160°C until the polymer started to precipitate from the melt. Shortly after partial precipitation, the stirring was terminated and the reaction was continued at about 160°C for about 2 hours. At the conclusion of the polymerization, the temperature was lowered below 120°C and excess monomer was removed under reduced pressure. The composition of the isolated polymer was verified using infrared and NMR spectroscopy.
**Micronization-** Each of the polymers of Examples I(a), I(b) and I(c) were ground initially using a Knife-grinder (IKA, Staufen, Germany). They were then micronized in an Aljet Micronizer (Fluid Energy Aljet, Plumsteadsville, Pennsylvania, USA) using a pressurized dry nitrogen stream. Example I(a) had a mean particle diameter size of 24.84 µm by analysis in a Malvern Mastersizer/E (Malvern, Worcs., U.K.) using a volume distribution model and 200/5 cS silicone oil (Dow Corning, Seneffe, Belgium) as dispersant. Examples I(b) and I(c) had mean particle diameter sizes of 4.69 µm and 6.31 µm, respectively, after micronization.
**Purification/Sodium Salt Formation-** Fifty gram batches of Examples I (a) , I (b) , and I (c) were dispersed in 2L of acetone (Riedel de-Haen, Seelze, Germany) and placed in a sonicator (Branson Ultrasonics BV, Soest, The Netherlands) for about 30 minutes. During this time the dispersion was also homogenized at about 9,500 rpm using an Ultra-turrax T25 homogenizer (IKA, Staufen, Germany). After this sonication/ homogenization step the dispersion was centrifuged at about 5,000 rpm for about 30 minutes in a Sorvall centrifuge (Sorvall, Wilmington, Delaware, USA). The supernatant was discarded, the centrifuge cakes resuspended in fresh acetone, and the sonication/homogenization step repeated. Once the second centrifugation was complete, the supernatant was discarded and the cakes were re-suspended in deionized water. One final sonication/homogenization step was then carried out to remove any remaining acetone and the dispersion was once again centrifuged at about 5,000 rpm for about 30 minutes.

The centrifuge cakes were re-suspended in fresh deionized water and the pH of the dispersion was monitored. Sufficient volumes of 0.2M sodium carbonate solution were added in each case (with stirring) to raise the pH to between about pH 8 and about pH 9. The dispersions were allowed to stir for about 30 minutes before being vacuum-filtered over a Whatman no.1 (24 cm diameter) filter paper (Whatman Intl. Ltd., Maidstone, Kent, U.K.). The filter cakes were rinsed with further deionized water, frozen, and lyophilized in an Edwards SuperModulyo Lyophilizer (Edwards, Crawley, West Sussex, U.K.).

Purification was monitored by differential scanning calorimetry (DSC) using a TA DSC912S (TA Instruments, New Castle, Delaware, USA) with a heating rate of 10°C/min. The DSC thermograms obtained in each case did not show any endothermic peak for monomeric glycolide but showed endotherms at 176°C, 178°C, and 180°C for Examples I(a) , I(b), and I(c), respectively.

### Example II

### Preparation of Microparticulate Cation-Exchanger of Glycolide/Malic Acid Copolymer PGMA

The title microparticle was synthesized according to the method described in Example I(c) but using glycolide (2.586 mole, 300 g), anhydrous malic acid (0.172 mole, 23 g), and stannous octoate (0.2 M in toluene, 862ml, 0.172 m mole). Differential Scanning Calorimetry was used to determine the polymer melting temperature (Tm = 206°C).

The solid polymer was ground to achieve average particle diameter of about 125 µm using a Wiley mill. Further reduction of the particle size to about 5-10 µm diameter was achieved using a jet-mill receiving pressurized dry nitrogen. The resulting microparticles were rinsed with acetone to remove trace monomer and low molecular weight oligomers. The product was then dried under reduced pressure at 40°C until used. The average diameter of the dry microparticle was determined using a particle size analyzer.

### Example III

### Preparation, Micronization, and Purification of a Poly(glycolic acid) polymer initiated with Tartaric Acid (PGTA) for use as a Cation Exchanger (CE)

**Example III(a): 10/1 PGTA-** A 500 ml glass reactor was loaded with 264.65 g of glycolide (Purac Biochem, Arkelsedijk, The Netherlands) and 34.22 g of L-Tartaric acid (Riedel de-Haen, Seelze, Germany). The tartaric acid had been further dried over silica gel (Fisher Scientific, Loughborough, Leics., U.K.) in an Abderhalden apparatus (Aldrich, St. Louis, MO). The reactor was immersed in an oil bath at about 40°C and put under vacuum (0.04 mbar) for about 30 minutes. The bath was then lowered and it's temperature raised to about 110°C. Once this temperature was reached the reactor was placed under an atmosphere of oxygen-free nitrogen and re-immersed. The contents were stirred at about 100 rpm using a Heidolph stirrer (Heidolph Elektro GmbH, Kelheim, Germany). Once the reactor contents melted 1.14 ml of a 0.1M stannous 2-ethyl-hexanoate solution (Sigma, St. Louis, Missouri, USA) in toluene (Riedel de-Haen, Seelze, Germany) was added (stoichiometric ratio of 50 ppm). A vacuum was reapplied via a liquid nitrogen trap for about 30 seconds to remove toluene without significant removal of monomer. The oil bath temperature was then raised to about 120°C for about 5 minutes before further raising it to about 150°C. It was kept at this temperature for about 4 hours under constant mechanical stirring of about 100 rpm. The title polymer was obtained.
**Micronization**-Example III(a) was ground initially using a Knife-grinder (IKA, Staufen, Germany). It was then micronized in an Aljet Micronizer (Fluid Energy Aljet, Plumsteadsville, Pennsylvania, USA) using a pressurized dry nitrogen stream. This gave a mean particle diameter of 12.42 µm by analysis in a Malvern Mastersizer/E (Malvern, Worcs., U.K.) using a volume distribution model and 200/5 cS silicone oil (Dow Corning, Seneffe, Belgium) as dispersant.
**Purification/Sodium Salt Formation**-A 50 g batch of Example III(a) was dispersed in 2L of acetone (Riedel de-Haen) and placed in a sonicator (Branson Ultrasonics BV, Soest, The Netherlands) for about 30 minutes. During this time the dispersion was also homogenized at about 9,500 rpm using an Ultra-turrax T25 homogenizer (IKA, Staufen, Germany). After this sonication/homogenization step the dispersion was centrifuged at about 5,000 rpm for about 30 minutes in a Sorvall centrifuge (Sorvall, Wilmington, Delaware, USA). The supernatant was discarded, the centrifuge cakes resuspended in fresh acetone, and the sonication/homogenization step repeated. Once the second centrifugation was complete, the supernatant was discarded and the cakes were re-suspended in deionized water. One final sonication/homogenization step was then carried out to remove any remaining acetone and the dispersion was once again centrifuged at about 5,000 rpm for about 30 minutes.

The centrifuge cakes were resuspended in fresh deionized water and the pH of the dispersion was monitored. A sufficient volume of 0.2M sodium carbonate solution was added to raise the pH to between about pH 8 and about pH 9. The dispersion was allowed to stir for about 30 minutes before being vacuum-filtered over a Whatman no.1 (24 cm diameter) filter paper (Whatman Intl. Ltd., Maidstone, Kent, U.K.). The filter cake was rinsed with further deionized water, frozen, and lyophilized in an Edwards SuperModulyo Lyophilizer (Edwards, Crawley, West Sussex, U.K.).

Purification was monitored by DSC using a TA DSC912S (TA Instruments New Castle, Delaware, USA) with a heating rate of about 10°C/min. The DSC thermogram obtained did not show any endothermic peak for monomeric glycolide but showed an endotherm at 181°C.
**Example III(b): 15/1 PGTA-** The title polymer was synthesized according to the procedure described for Example I(c) but using glycolide (2.586 mole, 300 g), anhydrous tartaric acid (0.172 mole, 26.8 g) and stannous octoate (0.2 M in toluene, 862 ml, .0172 mmole). Differential Scanning Calorimetry was used to determine the polymer melting temperature (Tm = 204°C).

The solid polymer was ground to achieve average particle diameter of about 125 µm using a Wiley mill. Further reduction of the particle size to about 5-10 µm diameter was achieved using a jet-mill receiving pressurized dry nitrogen. The resulting microparticles were rinsed with acetone to remove trace amounts of monomer and low molecular weight oligomers. The product was then dried under reduced pressure at about 40°C until used. The average diameter of the dry microparticle was determined using a particle size analyzer.

### Example IV

### Preparation of Polyglycolide-based Microparticulate Anion-Exchanger (AE-1)

The preparation of an anion-exchanger is achieved in two steps. First, low molecular weight polyglycolide is prepared using a similar procedure in Example I(c), but using the following polymerization charge: glycolide (1 mole, 116 g), 1,3 propanediol as an initiator (30 mmole, 2.22 g) and stannous octoate (0.03 mmole). The size reduction and purification of the polymer are then conducted as also described in Example I(c). In the second step, the practically non-ionic microparticles are incubated in hot dilute solution (∼80°C) of a diamine, for example hexanediamine of known concentration in dioxane under argon. The concentration of the diamine in dioxane is determined by acidimetry. When the reaction practically ceases to take place, the amidated microparticles are separated by filtration, rinsed with dioxane, and dried under reduced pressure. The binding capacity of the anion-exchanger (amidated particles) is determined by (1) elemental analysis for nitrogen and (2) extent of binding to Naproxen by measuring the extent of drug removed from a dilute solution using HPLC. The latter is confirmed by release of the immobilized Naproxen with a dilute sodium hydroxide solution of known concentration.

### Example V

### Preparation of Poly(lactide co-glycolide) copolymers initiated with propanediol (PLGPD) for use as encasing materials

**Example V(a): 75/25 P(1)LGPD-** A 500 ml glass reactor was loaded with 235.01 g of l-lactide (Purac Biochem, Arkelsedijk, The Netherlands), 63.09 g of glycolide (Purac Biochem, Arkelsedijk, The Netherlands) and 1.90 g of propanediol (Riedel de-haen, Seelze, Germany) and then 3.96 ml of a 0.1M stannous 2-ethyl-hexanoate solution (Sigma, St. Louis, Missouri, USA) in toluene (Riedel de-haen, Seelze, Germany) was added (stoichiometric ratio of 200 ppm). After drying under vacuum for about one hour to remove the toluene, the reactor was placed under an atmosphere of oxygen-free nitrogen and immersed in an oil bath preheated at about 160°C. The reactor contents were stirred at about 100 rpm with a Heidolph stirrer (Heidolph Elektro GmbH, Kelheim, Germany). Once the contents had melted the temperature was increased to about 180°C and maintained at this level for about 3 hours. An amorphous copolymer was obtained. The copolymer was found to have a molecular weight (MW) of about 12,500 g/mol by gel permeation chromatography (GPC) on a Waters 510 Pump, Waters 410 Differential Refractometer (Waters, Milford, Massachusetts, USA) with light-scattering detection on a Wyatt Minidawn Light Scattering Detector (Wyatt Technology Corporation, Santa Barbara, California, USA).
**Example V(b): 90/10 P(1)LGPD-** The title product was synthesized according to the procedure of Example V(a) but using 274.31 g of l-lactide, 24.55 g of glycolide, 1.14 g of propanediol and 3.89 ml of a 0.1M stannous 2-ethyl-hexanoate solution in toluene (stoichiometric ratio of 200 ppm). A crystalline copolymer was obtained. The copolymer was found to have a molecular weight of about 20,780 g/mol by GPC.
**Example V(c): 90/10 P(d,l)LGPD-** The title product was obtained by following the procedure of Example V(a) but using 274.31 g of d,l-lactide, 24.55 g of glycolide, 1.14 g of propanediol and 3.86 ml of a 0.1M stannous 2-ethyl-hexanoate solution in toluene (stoichiometric ratio of 200 ppm). An amorphous copolymer was obtained. The copolymer was found to have a molecular weight of about 20,650 g/mol by GPC.

### Example V(d): Poly(l-lactide co-d,l-lactide) copolymer initiated with propanediol (PLGPD) for use as coating material, 80/20 P(1)L(d,l)LPD

The title product was obtained by following the procedure of Example V(a) but using 239.09g of l-lactide, 59.77g of d,l-lactide (Purac Biochem, Arkelsedijk, The Netherlands) and 1.14g of propanediol and 3.96 ml of a 0.1M stannous 2-ethyl-hexanoate solution in toluene was added (stoichiometric ratio of 200ppm). An amorphous copolymer was obtained. The copolymer was found to have a molecular weight (Mw) of 22,320 g/mol by GPC. It showed a glassy transition at 48°C by DSC.
**Purification-** Examples V(a), V(b), and V(c) were each washed by nebulization of a 30% (W/W) solution in acetonitrile (Labscan, Dublin, Ireland) at 8 ml/min into deionized water cooled to about 2°C in a 6L jacketed reactor linked to a circulation bath and stirred at about 350 rpm with a Heidolph stirrer (Heidolph Elektro GmbH, Kelheim, Germany). The solutions were fed to a Vibra-Cell VC 50 Atomization nozzle (Bioblock, Illkirch, France) using a Masterflex pump (Cole Parmer Instrument Co., Niles, Illinois, USA) and nebulization was achieved using a sonication frequency of 12 kHz. The dispersions obtained were filtered over Whatman No.1 (24 cm diameter) filter papers (Whatman Intl. Ltd., Maidstone, Kent, U.K.) and the filter cakes were rinsed with deionized water, frozen, and lyophilized in an Edwards SuperModulyo Lyophilizer (Edwards, Crawley, West Sussex, U.K.).

Purity was confirmed by DSC using a TA DSC912s (TA Instruments, New Castle, Delaware, USA) with a heating rate of 10°C/min which showed glass transitions (Tg) at 44°C, 49°C, 45°C and 48°C for Examples V(a), V(b), V(c) and V(d), respectively.

### Example VI

**Preparation of Peptide-Loaded Cation Exchangers Example VI(a): Loading with Peptide A (p-Glu-His-Trp-Ser-Tyr-D-Trp-Leu-Arg-Pro-Gly-NH**_{**2**}**, an LHRH analog)-** Four grams of each of the sodium salts of Examples I (a) , I (b) , I (c) and II(a) were dispersed in solutions containing 1.33 g of the free-base of Peptide A (Kinerton Ltd., Dublin, Ireland) dissolved in 70 ml of deionized water. The dispersions were incubated at room temperature with stirring for about 2 hours before filtering over a 9 cm diameter Whatman No.1 filter paper (Whatman Intl. Ltd., Maidstone, Kent, U.K.). The filter cakes were rinsed with further deionized water, frozen, and lyophilized in an Edwards SuperModulyo (Edwards, Crawley, West Sussex, U.K.). Samples were then analyzed for nitrogen by elemental analysis to determine the amount of Peptide A bound. The following results were obtained :

| **Example** | **CE** **Ex.-#** | **CE** **Polymer** | **wt. % Peptide A** **Bound** |
|---|---|---|---|
| VI(a) ( i) | I(a) | 7/1 PGCA | 24.52% |
| VI(a) (ii) | I(b) | 10/1 PGCA | 12.60% |
| VI(a) (iii) | I(c) | 15/1 PGCA | 19.29% |
| VI(a) (iv) | III(a) | 10/1 PGTA | 17.60% |

**Example VI (b) : Loading with Peptide B (H-β-D-Nal-Cys-Tyr-D-Trp-Lys-Val-Cys-Thr-NH**_{**2**}**, the two Cys are bonded by a disulfide bond, a somatostatin analogue)-** Following the procedure of Example VI(b) and using 4 g of each of the sodium salts of Examples I(a), I(b), I(c) and II(a) and 1.33 g of the free-base of Peptide B (Kinerton Ltd., Dublin, Ireland) bound microparticles of Examples I(a), I(b) and I(c) with peptide B immobilized thereon were obtained. Samples were analyzed for nitrogen content by elemental analysis to determine the amount of Peptide B bound. The results obtained are shown below :

| **Example** | **CE** **Ex.-#** | **CE** **Polymer** | **wt. % Peptide B** **Bound** |
|---|---|---|---|
| VI(b) ( i) | I(a) | 7/1 PGCA | 25.20% |
| VI(b) (ii) | I(b) | 10/1 PGCA | 13.10% |
| VI(b) (iii) | I(c) | 15/1 PGCA | 19.64% |
| VI(b) (iv) | III(a) | 10/1 PGTA | 14.23% |

### Example VII

### Preparation of Encased Polypeptide-Loaded Cation Exchangers by Nebulization

Polypeptide-loaded cation exchangers were dispersed in acetonitrile (Labscan, Dublin, Ireland) solutions of encasing copolymers, indicated below. This dispersal was achieved by homogenizing with an Ultra-turrax T25 (IKA, Staufen, Germany) at about 9,500 rpm for about 5 minutes. The concentration of the encasing copolymer/ acetonitrile solutions ranged from 12.5% to 25% (W/W) and the ratio of encasing copolymer to polypeptide-loaded cation exchanger ranged from 1:1 to 1.3:1 by weight.

After dispersal, the dispersion was fed to a Vibra-Cell VC50 atomization nozzle (Bioblock, Illkirch, France) with a sonication frequency of 16 kHz using a ceramic piston pump (FMI, Oyster Bay, N.Y., USA) set at 2ml/min flow rate. Upon reaching the nozzle the dispersion was nebulized into isopropyl alcohol (IPA) (Labscan, Dublin, Ireland) cooled to about -80°C by the addition of dry-ice pellets (A.I.G., Dublin, Ireland). The IPA acted as a collecting non-solvent and was stirred at about 300 rpm using a Heidolph stirrer (Heidolph Elektro GmbH, Kelheim, Germany). Once nebulization was complete the entire dispersion was allowed to thaw to a temperature between about 10°C and about room temperature. The encased microparticles were then recovered by vacuum filtration over a Whatman No.1 filter paper (Whatman Intl. Ltd., Maidstone, Kent, U.K.). The filter cake was rinsed with deionized water, frozen and lyophilized in an Edwards SuperModulyo lyophilizer (Edwards, Crawley, West Sussex, U.K.). The resulting encased microparticles were analyzed for size using the Malvern Mastersizer/E (Malvern, Worcs., U.K.) and 1% Tween 20 in water as a dispersant. The encased microparticles were also analyzed for nitrogen content by elemental analysis to determine peptide content.
The table below represents the various encasing experiments carried out :

| Ex. # | Peptide Loaded CE: Ex-# | Encasing Copolymer Ex-# | Conc. (W/W) of Encasing Copolymer in Acetonitrile | Encasing Copolymer : Peptide-loaded CE | Mean Particle Diameter | wt.% Peptide Loading |
|---|---|---|---|---|---|---|
| VII(a) | VI(a) (ii) | V(a) | 24.31% | 1:1 | 122.14µm | 5.38% Peptide A |
| VII(b) | VI(a) (ii) | V(b) | 22.41% | 1:1 | 120.15µm | 6.38% Peptide A |
| VII(c) | VI(a) (iii) | V(b) | 12.5% | 1:1 | 79.30µm | 7.76% Peptide A |
| VII(d) | VI(a) (iii) | V(c) | 12.5% | 1:1 | 77.85µm | 8.93% Peptide A |
| VII(e) | VI(a) (iv) | V(c) | 14.95% | 1:1 | 136.74µm | 8.75% Peptide A |
| VII(f) | VI(a) (i) | V(c) | 14.92% | 1.27:1 | 80.59µm | 10.31% Peptide A |
| VII(g) | VI(b) (ii) | V(a) | 25.37% | 1:1 | 140.58µm | 2.63% Peptide 'B |
| VII(h) | VI(b) (ii) | V(b) | 20% | 1.15:1 | 96.77µm | 5.98% Peptide B |
| VII(i) | VI(b) (iii) | V(b) | 12.5% | 1:1 | 102.56µm | 7.69% Peptide B |
| VII(j) | VI(b) (iii) | V(c) | 12.5% | 1:1 | 83.72µm | 7.90% Peptide B |
| VII(k) | VI(b) (iv) | V(c) | 14.95% | 1:1 | 135.14µm | 6.69% Peptide B |
| VII(l) | VI(b) (i) | V(c) | 14.92% | 1.26:1 | 123.18µm | 10.11% Peptide B |

All samples were sieved over a 180 µm sieve (Bioblock, Illkirch, France) prior to *in vivo* and/or *in vitro* testing.

A bound microparticle or encased microparticle can be tested *in vitro* to assess the release rate of a bound peptide or bound protein by the following method. An aliquot of a bound microparticle or encased microparticle having a mass of about 50 mg is placed in a continuous flow-cell system where a buffered phosphate solution at about pH 7.2 and at about 37°C flow across the entire mass of the bound microparticles or encased microparticles at a rate of about 45 ml/hr. Samples of the buffer containing the released drug are collected at about 4°C and analyzed for the peptide or protein concentrations at 1- or 2-day intervals. The release profile of each microparticle is determined over a period of 2 weeks.

A bound microparticle or encased microparticle can be tested to assess the release rate of a bound peptide or bound protein in an in vivo system by the following method. Samples are administered to male Wistar rats (Bioresources, Trinity College, Dublin, Ireland) by intramuscular injection to the thigh. The suspension medium consists of 3% carboxymethylcellulose and 1% Tween 20 in saline solution. For Peptide A-loaded samples the effective equivalent dose is 40 µg/Kg/day. The dose for Peptide B-loaded samples is 1 mg/Kg/day. Samples are taken by cardiac puncture and the plasma peptide levels are monitored by radioimmunoassays (RIA) specific for Peptide A and Peptide B. In the case of Peptide A-loaded samples (Peptide A is an LHRH analog), a testosterone RIA is also used to monitor testosterone suppression. As an alternative to the suspension medium, gel-formers can be used in certain cases. The results are shown in Tables A and B, below.

**Table A**

| **Peptide A** **Examples** | **Peptide A (>150 pg/ml)** **Days** | **Testosterone (<1ng/ml)** **Days** |
|---|---|---|
| VII (a) | 20 | 21 |
| VII (b) | 10 | 10 |
| VII (c) | 2 | 11 |
| VII (d) | 2 | 11 |
| VII (e) | 2 | 13 |
| VII (f) | 2 | 16 |
| VII (a) in gel-former | 25 | 44 |

**Table B**

| **Peptide B** **Examples** | **Peptide B (>1000 pg/ml)** **Days** |
|---|---|
| VII (g) | Not tested |
| VII (h) | Not tested |
| VII (i) | Not tested |
| VII (j) | 15 |
| VII (k) | 10 |
| VII (l) | 10 |

### Example VIII

### Example VIII(a): Nebulization using acetonitrile as solvent and room temperature IPA as non-solvent

About 1.06 g of the cation exchanger of Example I(c) (not bound to polypeptide) was dispersed in a 25.24% (W/W) solution of encasing copolymer of Example V(a) in acetonitrile (Labscan, Dublin, Ireland) such that the ratio of cation exchanger to encasing copolymer was about 1.03:1 by weight. This dispersal was achieved by homogenizing with an Ultra-turrax T25 (IKA, Staufen, Germany) at about 9,500rpm for about 5 minutes.

After dispersal, the dispersion was fed to a Vibra-Cell VC50 atomization nozzle (Bioblock, Illkirch, France) with a sonication frequency of 16kHz using a ceramic piston pump (FMI, Oyster Bay, N.Y., U.S.A.) set at 2ml/min flowrate. Upon reaching the nozzle the dispersion was nebulized into IPA (Labscan, Dublin, Ireland) at room temperature (17 to 22°C). This IPA acted as a collecting non-solvent and was stirred at about 300rpm using, a Heidolph stirrer (Heidolph Elektro GmbH, Kelheim, Germany). Once nebulization was complete the dispersion was left to stir for about another 60 minutes at room temperature before the encased particles were recovered by vacuum filtration over a Whatman No. 1 filter paper (Whatman Intl. Ltd., Maidstone, Kent, U.K.). The filter cake was rinsed with deionized water, frozen and lyophilized in an Edwards SuperModulyo lyophilizer (Edwards, Crawley, West Sussex, U.K.). The resulting particles were analyzed for particle size using the Malvern Mastersizer/E (Malvern, Worcs., U.K.) and 1% Tween 20 in water as a dispersant. The resulting particles had a mean particle size (d(0.5)) of 84.75µm.

### Example VIII(b): Nebulization using Ethyl Acetate as solvent and room-temperature IPA as non-solvent

The nebulization was carried out substantially according to the procedure of Example VIII(a) but using about 0.99g of cation exchanger of Example I(c) (not bound to polypeptide) dispersed in a 24.88% (W/W) solution of encasing copolymer of Example V(a) in ethyl acetate (Riedel-de Haen, Seelze, Germany) such that the ratio of cation exchanger to encasing copolymer was about 0.96:1 by weight. The resulting particles had a mean particle size (d(0.5)) of 100.56µm.

### Example VIII(c): Nebulization using Ethyl Acetate as solvent and a higher frequency probe

About 1.02g of cation exchanger of Example I(c) (not bound to polypeptide) was dispersed in a 15.14% (W/W) solution of encasing copolymer of Example V(a) in ethyl acetate (Riedel-de Haen) such that the ratio of cation exchanger to encasing copolymer was about 1.05:1 by weight. This dispersal was achieved by homogenizing with an Ultra-turrax T25 (IKA, Staufen, Germany) at about 9,500 rpm for about 5 minutes.

After dispersal, the dispersion was fed to a Martin Walter 400 GSIP nebulizer (Sodeva, Le Bouget du Lac, France) with an ultrasonic frequency setting of about 34.6kHz using a ceramic piston pump (FMI, Oyster Bay, N.Y., U.S.A.) set at 5ml/min flow rate. Upon reaching the nozzle the dispersion was nebulized into IPA (Labscan, Dublin, Ireland) cooled to about -77° by the addition of dry-ice pellets (A.I.G., Dublin, Ireland). This IPA acted as a collecting non-solvent and was stirred at 300rpm using a Heidolph stirrer (Heidolph Elektro GmbH, Kelheim, Germany). Once nebulization was complete the coated particles were recovered by vacuum filtration over a Whatman No. 1 filter paper (Whatman Intl. Ltd., Maidstone, Kent, U.K.). The filter cake was rinsed with deionized water, frozen and lyophilized in an Edwards SuperModulyo lyophilizer (Edwards, Crawley, West Sussex, U.K.). The resulting particles were analyzed for particle size using the Malvern Masterizer/E (Malvern, Worcs., U.K.) and 1% Tween 20 in water as a dispersant. The resulting particles had a mean particle size (d(0.5)) of 95.69µm.

### Example IX

### Binding to cation exchanger and subsequent encasing of Somatostatin Analog Peptides C and D

### Example IX(a): Loading with peptide C

About 1.01g of the sodium salt of Example I(c) dispersed in a solution containing 0.25g of the free base of peptide C, which has the structure N-hydroxyethylpiperazinyl-acetyl-D-Phe-Cys-Tyr-D-Trp-Lys-Abu-Cys-Thr-NH₂ where the two Cys residues are bonded by a disulfide bond (Kinerton Ltd., Dublin, Ireland), dissolved in 40 ml deionized water. The dispersion was incubated with stirring for about 2 hours before filtering over a 9 cm diameter Whatman No. 1 filter paper (Whatman Intl. Ltd:, Maidstone, Kent, U.K.). The filter cake was rinsed with further deionized water, frozen, and lyophilized in an Edwards SuperModulyo (Edwards, Crawley, West Sussex. U.K.). The sample was then sent for nitrogen analysis to determine the amount of peptide bound, 20.21%.

### Example IX(b): Loading with peptide D

Using the procedure of Example IX(a) but using about 2.04 g of the sodium salt of Example I(c) dispersed in a solution containing 0.51g of the free base of peptide D, which has the structure N-hydroxyethylpiperazinyl-ethylsulfonyl-Phe-Cys-Tyr-D-Trp-Lys-Abu-Cys-Thr-NH₂ where the two Cys residues are bonded by a disulfide bond (Kinerton Ltd., Dublin, Ireland), dissolved in 80 ml deionized water. The sample was then sent for nitrogen analysis to determine the amount of peptide bound, 19.53%.

### Example X

The bound microparticles of Examples IX(a) and IX(b) were encased as described in Example VII yielding the following results:

| Ex. No. | Peptide-loaded CE | Coating copolymer | Conc. (W/W) of coating copolymer in acetonitrile | Coating copolymer:Peptide loaded CE | Mean Particle Size (µm) | Wt. % Peptide Loading |
|---|---|---|---|---|---|---|
| X (a) | IX (a) | V (c) | 12.51% | 1:1 | 83.33 | 9.48% |
| X (b) | IX (b) | V (c) | 12.48% | 0.98:1 | 72.15 | 8.87% |
| X (c) | IX (b) | V (d) | 12.35% | 0.98:1 | 86.03 | 6.74% |

### Example XI

### Preparation of a Gel-Former Formulation

Encased microparticles of Example VII(a) (0.3 g) were mixed with a liquid gel former (2.0 mL of a 50/50 mixture of component "A" of Example I and component C of Example III, both of which are disclosed in U.S. Patent No. 5,612,052) in a 5 mL syringe barrel using a mechanical micromixer at about 20 rpm for about 10 minutes. The gel-former was presterilized by dry heat and the mixing was conducted using a sterilized stirrer in a laminar flow hood. The formulation was extruded from the 5 mL syringe (after introducing the plunger) into a smaller syringe which is intended for use in administering the formulation. The uniformity of the formulation was checked using optical microscopy. The small syringes were assembled for storage in a dry package and kept at about 4°C until use.

## Claims

1. A bound microparticle comprising an absorbable heterochain polymer core and one or more peptide, one or more protein or a combination thereof ionically immobilized on said absorbable heterochain polymer core,
wherein each peptide is independently selected from the group consisting of growth hormone releasing peptide (GHRP), luteinizing hormone-releasing hormone (LHRH), somatostatin, bombesin, gastrin releasing peptide (GRP), calcitonin, bradykinin, galanin, melanocyte stimulating hormone (MSH), growth hormone releasing factor (GRF), amylin, tachykinins, secretin, parathyroid hormone (PTH), enkaphelin, endothelin, calcitonin gene releasing peptide (CGRP), neuromedins, parathyroid hormone related protein (PTHrP), glucagon, neurotensin, adrenocorticothrophic hormone (ACTH), peptide YY (PYY), glucagon releasing peptide (GLP), vasoactive intestinal peptide (VIP), pituitary adenylate cyclase activating peptide (PACAP), motilin, substance P, neuropeptide Y (NPY), TSH and analogs and fragments thereof or a pharmaceutically acceptable salt thereof; and
wherein each protein is independently selected from the group consisting of growth hormone, erythropoietin, granulocyte-colony stimulating factor, granulocyte-macrophage-colony stimulating factor and interferons.

2. A bound microparticle according to claim 1 wherein said peptide, protein or a combination thereof or a pharmaceutically acceptable salt thereof comprises 0.1% to 30% of the total mass of the bound microparticle.

3. A bound microparticle according to claim 2 wherein said absorbable heterochain polymer core comprises glycolate units.

4. A bound microparticle according to claim 3 wherein the absorbable heterochain polymer core further comprises citrate residues, tartrate residues or malate residues.

5. A bound microparticle according to claim 4 wherein the ratio of glycolate units to said citrate residue, to said tartrate residue or to said malate residue is about 7-1 to about 20-1.

6. A bound microparticle according to claim 3 wherein said glycolate units terminate with a carboxyl moiety, or an amine moiety.

7. An encased microparticle comprising one or more of a bound microparticle encased within an absorbable encasing polymer,
wherein said bound microparticle comprises an absorbable heterochain polymer core and one or more peptide, one or more protein or a combination thereof ionically immobilized on said absorbable heterochain polymer core,
where each peptide is independently selected from the group consisting of growth hormone releasing peptide (GHRP), luteinizing hormone-releasing hormone (LHRH), somatostatin, bombesin, gastrin releasing peptide (GRP), calcitonin, bradykinin, galanin, melanocyte stimulating hormone (MSH), growth hormone releasing factor (GRF), amylin, tachykinins, secretin, parathyroid hormone (PTH), enkaphelin, endothelin, calcitonin gene releasing peptide (CGRP), neuromedins, parathyroid hormone related protein (PTHrP), glucagon, neurotensin, adrenocorticothrophic hormone (ACTH), peptide YY (PYY), glucagon releasing peptide (GLP), vasoactive intestinal peptide (VIP), pituitary adenylate cyclase activating peptide (PACAP), motilin, substance P, neuropeptide Y (NPY), TSH and analogs and fragments thereof or a pharmaceutically acceptable salt thereof;
where each protein is independently selected from the group consisting of growth hormone, erythropoietin, granulocyte-colony stimulating factor, granulocyte-macrophage-colony stimulating factor and interferons; and
where said absorbable heterochain polymer core comprises glycolate units.

8. An encased microparticle according to claim 7 wherein said peptide, protein or combination thereof or pharmaceutically acceptable salt thereof comprises 0.1% to 30% of the total mass of the bound microparticle, and where said absorbable heterochain polymer core further comprises citrate residues, tartrate residues or malate residues.

9. An encased microparticle according to claim 8 wherein the ratio of glycolate units to citrate residues, to tartrate residues or to malate residues is about 7-1 to about 20-1 and said glycolate units terminate with a carboxyl moiety or an amine moiety.

10. An encased microparticle according to claim 9 wherein said absorbable encasing polymer comprises
(a) l-lactide based units and glycolide based units,
(b) d,l-lactide based units and glycolide based units,
(c) d,l-lactide based units or
(d) l-lactide based units and d,l-lactide based units.

11. An encased microparticle according to claim 10 wherein
the ratio of 1-lactide based units to glycolide based units is about 75-25 to about 90-10; or
the ratio of l-lactide based units to d,l-lactide based units is about 80-20; or
the ratio of d,l-lactide based units to glycolide based units is about 75-25 to about 90-10.

12. An encased microparticle according to claim 9 wherein the absorbable encasing polymer constitutes 5 to 70% of the total mass of the encased microparticle.

13. An encased microparticle according to claim 12 wherein the absorbable encasing polymer constitutes 20-60%, and preferably 20-50% of the total mass of the encased microparticle.

14. A pharmaceutical composition comprising the bound microparticles according to claim 1 and a pharmaceutically acceptable carrier.

15. A pharmaceutical composition comprising the bound microparticles according to claim 1, a non-aqueous absorbable gel-forming liquid polyester and optionally a pharmaceutically acceptable carrier.

16. A pharmaceutical composition comprising the encased microparticles according to claim 7 and a pharmaceutically acceptable carrier.

17. A pharmaceutical composition comprising the encased microparticles according to claim 7, a non-aqueous absorbable gel-forming liquid polyester and optionally a pharmaceutically acceptable carrier.

18. A bound microparticle according to claim 3 wherein said absorbable heterochain polymer core further comprises citrate residues and said peptide is an LHRH analog or a somatostatin analog

19. A bound microparticle according to claim 18 wherein the ratio of glycolate units to citrate residues of the absorbable heterochain polymer core is about 7-1 to about 20-1 and where the peptide is said LHRH analog which is p-Glu-His-Trp-Ser-Tyr-D-Trp-Leu-Arg-Pro-Gly-NH₂.

20. A bound microparticle according to claim 18 wherein the ratio of glycolate units to citrate residues is about 7-1 to about 20-1 and the peptide is said somatostatin analog which is H-β-D-Nal-Cys-Tyr-D-Trp-Lys-Val-Cys-Thr-NH₂ where the two Cys residues are bonded by a disulfide bond, N-hydroxyethylpiperazinyl-acetyl-D-Phe-Cys-Tyr-D-Trp-Lys-Abu-Cys-Thr-NH₂ where the two Cys residues are bonded by a disulfide bond or N-hydroxyethylpiperazinyl-ethylsulfonyl-Phe-Cys-Tyr-D-Trp-Lys-Abu-Cys-Thr-NH₂ where the two Cys residues are bonded by a disulfide bond.

21. A bound microparticle according to claim 3 wherein the absorbable heterochain polymer core further comprises tartrate residues and the peptide is an LHRH analog or a somatostatin analog.

22. A bound microparticle according to claim 21 wherein the ratio of glycolate units to tartrate residues is about 7-1 to about 20-1 and the peptide is said LHRH analog which is p-Glu-His-Trp-Ser-Tyr-D-Trp-Leu-Arg-Pro-Gly-NH₂.

23. A bound microparticle according to claim 21 wherein the ratio of glycolate units to tartrate residues is about 7-1 to about 20-1 and the peptide is said somatostatin analog which is H-β-D-Nal-Cys-Tyr-D-Trp-Lys-Val-Cys-Thr-NH₂, where the two Cys residues are bonded by a disulfide bond, N-hydroxyethylpiperazinyl-acetyl-D-Phe-Cys-Tyr-D-Trp-Lys-Abu-Cys-Thr-NH₂ where the two Cys residues are bonded by a disulfide bond or N-hydroxyethylpiperazinyl-ethylsulfonyl-Phe-Cys-Tyr-D-Trp-Lys-Abu-Cys-Thr-NH₂ where the two Cys residues are bonded by a disulfide bond.

24. An encased microparticle comprising one or more bound microparticles according to claim 18 or 21 encased within an absorbable encasing polymer which comprises
(a) l-lactide based units and glycolide based units,
(b) d,l-lactide based units and glycolide based units,
(c) d,l-lactide based units or
(d) l-lactide based units and d,l-lactide based units.

25. An encased microparticle according to claim 24, when dependent on claim 18, wherein the ratio of glycolate units to citrate residues of the absorbable polymer core is about 7-1 to about 20-1, the peptide is said LHRH analog which is p-Glu-His-Trp-Ser-Tyr-D-Trp-Leu-Arg-Pro-Gly-NH₂ and where the ratio of:
(a) l-lactide based units to glycolide based units is about 75-25 to about 90-10,
(b) d,l-lactide based units to glycolide based units is about 75-25 to about 90-10 and
(c) l-lactide based units to d,l-lactide based units is about 80-20.

26. An encased microparticle according to claim 24, when dependent on claim 21, wherein the ratio of glycolate units to tartrate residues of the absorbable polymer core is about 7-1 to about 20-1, the peptide is said LHRH analog which is p-Glu-His-Trp-Ser-Tyr-D-Trp-Leu-Arg-Pro-Gly-NH₂ and where the ratio of:
(a) l-lactide based units to glycolide based units is about 75-25 to about 90-10,
(b) d,l-lactide based units to glycolide based units is about 75-25 to about 90-10 and
(c) l-lactide based units to d,l-lactide based units is about 80-20.

27. An encased microparticle according to claim 24, when dependent on claim 18, wherein the ratio of glycolate units to citrate residues of the absorbable polymer core is about 7-1 to about 20-1, the peptide is said somatostatin analog which is H-β-D-Nal-Cys-Tyr-D-Trp-Lys-Val-Cys-Thr-NH₂ where the two Cys residues are bonded by a disulfide bond, N-hydroxyethylpiperazinyl-acetyl-D-Phe-Cys-Tyr-D-Trp-Lys-Abu-Cys-Thr-NH₂ where the two Cys residues are bonded by a disulfide bond or N-hydroxyethylpiperazinyl-ethylsulfonyl-Phe-Cys-Tyr-D-Trp-Lys-Abu-Cys-Thr-NH₂ where the two Cys residues are bonded by a disulfide bond;
and where the ratio of:
(a) l-lactide based units to glycolide based units is about 75-25 to about 90-10,
(b) d,l-lactide based units to glycolide based units is about 75-25 to about 90-10 and
(c) l-lactide based units to d,l-lactide based units is about 80-20.

28. An encased microparticle according to claim 24, when dependent on claim 21, wherein the ratio of glycolate units to tartrate residues of the absorbable polymer core is about 7-1 to about 20-1, the peptide is said somatostatin analog which is H-β-D-Nal-Cys-Tyr-D-Trp-Lys-Val-Cys-Thr-NH₂ where the two Cys residues are bonded by a disulfide bond, N-hydroxyethylpiperazinyl-acetyl-D-Phe-Cys-Tyr-D-Trp-Lys-Abu-Cys-Thr-NH₂ where the two Cys residues are bonded by a disulfide bond or N-hydroxyethylpiperazinyl-ethylsulfonyl-Phe-Cys-Tyr-D-Trp-Lys-Abu-Cys-Thr-NH₂ where the two Cys residues are bonded by a disulfide bond;
and where the ratio of:
(a) 1-lactide based units to glycolide based units is about 75-25 to about 90-10,
(b) d,l-lactide based units to glycolide based units is about 75-25 to about 90-10 and
(c) 1-lactide based units to d,l-lactide based units is about 80-20.

29. A process for making an encased microparticle according to claim 7 comprising the step of encasing a bound microparticle with an absorbable encasing polymer.

30. A process according, to claim 29 wherein a dispersion of said bound microparticles in a solution comprising said absorbable encasing polymer and a solvent is dropped onto a pre-cooled medium, where said medium is not a solvent of said absorbable encasing polymer.

31. A process according to claim 30 wherein the solution of the absorbable encasing polymer consists of about 5% to 30% of the absorbable encasing polymer, the pre-cooled medium is an alcohol having two or more carbon atoms and the temperature of the medium is room temperature to about -80°C.

32. A process according to claim 31 wherein the temperature of the pre-cooled medium is about -60°C to -80°C and the medium is isopropyl alcohol.

33. A process for making an encased microparticle according to claim 7 comprising the step of encasing a bound microparticle with an absorbable encasing polymer using an emulsion technique.

## Patentansprüche

1. Gebundenes Mikroteilchen, das einen absorhierbaren Heterokettenpolymerkern und ein oder mehrere Peptid(e), ein oder mehrere Protein(e) oder eine Kombination derselben ionisch immobilisiert auf dem absorbierbaren Heterokettenpolymerkern umfasst,
wobei jedes Peptid unabhängig ausgewählt ist aus der Gruppe bestehend aus Wachstumshormon freisetzendem Peptid (GHRP), luteinisierendes Hormon freisetzendem Hormon (LHRH), Somatostatin, Eombesin, Gastrin freisetzendem Peptid (GRP), Calcitonin, Bradykinin, Galanin, Melanozyten stimulierendem Hormon (MSH), Wachstumshormon freisetzendem Faktor (GRF), Amylin, Tachykininen, Sekretin, Parathyroidhormon (PTH) , Enkephalin, Endothelin, Calcitonin-Gen freisetzendem Peptid (CGRP), Neuromedinen, Parathyroidhormon verwandtem Protein (PTHrP), Glukagon, Neurotensin, adrenocorticotrophem Hormon (ACTH), Peptid YY (PYY), Glukagon freisetzendem Peptid (GLP), vasoaktivem Intestinalpeptid (VIP), pituitärem Adenylatcyclase aktivierenden Peptid (PACAP), Motilin, Substanz P, Neuropeptid Y (NPY), TSH und Analoga und Fragmente derselben oder einem pharmazeutisch akzeptablem Salz derselben; und
wobei jedes Protein unabhängig ausgewählt ist aus der Gruppe bestehend aus Wachstumshormon, Erythropoietin, Granulozyten-Kolonie stimulierendem Faktor, Granolozyt-Makrophage-Kolonie stimulierendem Faktor und Interferonen.

2. Gebundenes Mikroteilchen nach Anspruch 1, bei dem das Peptid, Protein oder eine Kombination derselben oder ein pharmazeutisch annehmbares Salz derselben 0,1 bis 30 % der Gesamtmasse des gebundenen Mikroteilchens umfasst.

3. Gebundenes Mikroteilchen nach Anspruch 2, bei dem der absorbierbare Heterokettenpolymerkern Glykolateinheiten umfasst.

4. Gebundenes Mikroteilchen nach Anspruch 3, bei dem der absorbierbare Heterokettenpolymerkern ferner citratreste, Tartratreste oder Malatreste umfasst.

5. Gebundenes Mikroteilchen nach Anspruch 4, bei dem das Verhältnis von Glykolateinheiten zu dem Citratrest, zu dem Tartratrest der zu dem Malatrest etwa 7-1 bis etwa 20-1 beträgt.

6. Gebundenes Mikroteilchen nach Anspruch 3, bei dem die Glykolateinheiten mit einem Carboxylanteil oder einem Aminanteil enden.

7. Ummanteltes Mikroteilchen, das ein oder mehrere gebundene Mikroteilchen ummantelt mit einem absorbierbaren Ummantelungspolymer umfasst, wobei
das gebundene Mikroteilchen einen absorbierbaren Heterokettenpolymerkern und ein oder mehrere Peptid(e), ein oder mehrere Protein(e) oder eine Kombination derselben ionisch immobilisiert auf dem absorbierbaren Heterokettenpolymerkern umfasst,
wobei jedes Peptid unabhängig ausgewählt ist aus der Gruppe bestehend aus Wachstumshormon freisetzendem Peptid (GHRP), luteinisierendes Hormon freisetzendem Hormon (LHRH), Somatostatin, Bombesin, Gastrin freisetzendem Peptid (GRP), Calcitonin, Bradykinin, Galanin, Melanozyten stimulierendem Hormon (MSH), Wachstumshormon freisetzendem Faktor (GRF), Amylin, Tachykininen, Sekretin, Parathyroidhormon (FTH), Enkephalin, Endothelin, Calcitonin-Gen freisetzendem Peptid (CGRP), Neuromedinen, Parathyroidhormon verwandtem Protein (PTHrP), Glukagon, Neurotensin, adrenocorticotrophem Hormon (ACTH), Peptid YY (PYY), Glukagon freisetzendem Peptid (GLP), vasoaktivem Intestinalpeptid (VIP), pituitärem Adenylatcyclase aktivierenden Peptid (PACAP), Motilin, Substanz P, Neuropeptid Y (NPY), TSH und Analoga und Fragmenten derselben oder einem pharmazeutisch akzeptablem Salz derselben; und
wobei jedes Protein unabhängig ausgewählt ist aus der Gruppe bestehend aus Wachstumshormon, Erythropoietin, Granulozyten-Kolonie stimulierendem Faktor, Granolozyt-Makrophage-Kolonie stimulierendem Faktor und Interferonen; und
wobei der absorbierbare Heterokettenpolymerkern Glykolateinheiten umfasst.

8. Ummanteltes Mikroteilchen nach Anspruch 7, bei dem das Peptid, Protein oder die Kombination oder das pharmazeutisch akzeptable Salz derselben 0,1 % bis 30 % der Gesamtmasse des gebundenen Mikroteilchens umfasst, und wobei der absorbierbare Heterokettenpolymerkern ferner Citratreste, Tartratreste oder Malatreste umfasst.

9. Ummanteltes Mikroteilchen nach Anspruch 8, bei dem das Verhältnis von Glykolateinheiten zu Citratresten, zu Tartratresten oder zu Malatresten etwa 7-1 bis etwa 20-1 beträgt und die Glykolateinheiten mit einem Carboxylanteil oder einem Aminanteil enden,

10. Ummanteltes Mikroteilchen nach Anspruch 9, bei dem das absorbierbare Ummantelungspolymer
(a) Einheiten auf l-Lactidbasis und Einheiten auf Glykolidbasis,
(b) Einheiten auf d,l-Lactidbasis und Einheiten auf Glykolidbasis,
(c) Einheiten auf d,l-Lactidbasis oder
(d) Einheiten auf l-Lactidbasis und Einheiten auf d,l-Lactidbasis
umfasst.

11. Ummanteltes Mikroteilchen nach Anspruch 10, bei dem
das Verhältnis von Einheiten auf l-Lactidbasis zu Einheiten auf Glykolidbasis etwa 75-25 bis etwa 90-10 beträgt; oder
das Verhältnis von Einheiten auf l-Lactidbasis zu Einheiten auf d,l-Lactidbasis etwa 80-20 beträgt; oder
das Verhältnis von Einheiten auf d,l-Lactidbasis zu Einheiten auf Glykolidbasis etwa 75-25 bis etwa 90-10 beträgt.

12. Ummanteltes Mikroteilchen nach Anspruch 9, bei dem das absorbierbare Ummantelungspolymer 5 bis 70 % der Gesamtmasse des ummantelten Mikroteilchens ausmacht.

13. Ummanteltes Mikroteilchen nach Anspruch 12, bei dem das absorbierbare Ummantelungspolymer 20 bis 60 % und vorzugsweise 30 bis 50 % der Gesamtmasse des ummantelten Mikroteilchens ausmacht.

14. Pharmazeutische Zusammensetzung, die die gebundenen Mikroteilchen gemäß Anspruch 1 und einen pharmazeutisch akzeptablen Träger umfassen.

15. Pharmazeutische Zusammensetzung, die die gebundenen Mikroteilchen gemäß Anspruch 1, einen nicht-wässrigen, absorbierbaren, gelbildenden, flüssigen Polyester und gegebenenfalls einen pharmazeutisch akzeptablen Träger umfasst.

16. Pharmazeutische Zusammensetzung, die die ummantelten Mikroteilchen gemäß Anspruch 7 und einen pharmazeutisch akzeptablen Träger umfasst.

17. Pharmazeutische Zusammensetzung, die die ummantelten Mikroteilchen gemäß Anspruch 7, einen nicht-wässrigen, absorbierbaren, gelbildenden, flüssigen Polyester und gegebenenfalls einen pharmazeutisch akzeptablen Träger umfasst.

18. Gebundenes Mikroteilchen nach Anspruch 3, bei dem der absorbierbare Heterokettenpolymerkern ferner Citratreste umfasst und das Peptid ein LHRH-Analogon oder ein Somatostatin-Analogon ist.

19. Gebundenes Mikroteilchen nach Anspruch 18, bei dem das Verhältnis von Glykolateinheiten zu Citratresten des absorbierbaren Heterokettenpolymerkerns etwa 7-1 bis etwa 20-1 beträgt und wobei das Peptid das LHRH-Analogon ist, das p-Glu-His-Trp-Ser-Tyr-D-Trp-Leu-Arg-Pro-Gly-NH₂ ist.

20. Gebundenes Mikroteilchen nach Anspruch 18, bei dem das Verhältnis von Glykolateinheiten zu Citratresten etwa 7-1 bis etwa 20-1 beträgt und das Peptid das Somatostatin-Analogon ist, das H-β-D-Nal-Cys-Tyr-D-Trp-Lys-Val-Cys-Thr-NH₂ ist, wobei die beiden Cys-Reste durch eine Disulfidbrücke verbunden sind, N-Hydroxyethylpiperazinyl-acetyl-D-Phe-Cys-Tyr-D-Trp-Lys-Abu-Cys-Thr-NH₂, wobei die beiden Cys-Reste durch eine Disulfidbrücke verbunden sind, oder N-Hydroxyethylpiperazinyl-ethylsulfonyl-Phe-Cys-Tyr-D-Trp-Lys-Abu-Cys-Thr-NH₂ ist, wobei die beiden Cys-Reste durch eine Disulfidbrücke verbunden sind.

21. Gebundenes Mikroteilchen nach Anspruch 3, bei dem der absorbierbare Heterokettenpolymerkern ferner Tartratreste umfasst und das Peptid ein LHRH-Analogon oder ein Somatostatin-Analogon ist.

22. Gebundenes Mikroteilchen nach Anspruch 21, bei dem das Verhältnis der Glykolateinheiten zu Tartratresten etwa 7-1 bis etwa 20-1 beträgt, und das Peptid das LHRH-Analogon ist, das p-Glu-His-Trp-Ser-Tyr-D-Trp-Leu-Arg-Pro-Gly-NH₂ ist.

23. Gebundenes Mikroteilchen nach Anspruch 21, bei dem das Verhältnis von Glykolateinheiten zu Tartratresten etwa 7-1 bis etwa 20-1 beträgt und das Peptid das Somatostatin-Analogon ist, das H-β-D-Na1-Cys-Tyr-D-Trp-Lys-Val-Cys-Thr-NH₂ ist, wobei die beiden cys-Reste durch eine Disulfidbrücke verbunden sind, N-Hydroxyethylpiperazinyl-acetyl-D-Phe-Cys-Tyr-D-Trp-Lys-Abu-Cys-Thr-NH₂, wobei die beiden Cys-Reste durch eine Disulfidbrücke verbunden sind, oder N-Hydroxyethylpiperazinyl-ethylsulfonyl-Phe-Cys-Tyr-D-Trp-Lys-Abu-Cys-Thr-NH₂ ist, wobei die beiden Cys-Reste durch eine Disulfidbrücke verbunden sind.

24. Ummanteltes Mikroteilchen, das ein oder mehrere gebundene Mikroteilchen gemäß Anspruch 18 oder 21 ummantelt mit einem absorbierbaren Ummantelungspolymer umfasst, das
(a) Einheiten auf 1-Lactidbasis und Einheiten auf Glykolidbasis,
(b) Einheiten auf d,l-Lactidbasis und Einheiten auf Glykolidbasis,
(c) Einheiten auf d,l-Lactidbasis oder
(d) Einheiten auf 1-Lactidbasis und Einheiten auf d,l-Lactidbasis
umfasst.

25. Ummanteltes Mikroteilchen nach Anspruch 24, wenn abhängig von Anspruch 18, bei dem das Verhältnis von Glykolateinheiten zu Citratresten in dem absorbierbaren Polymerkern etwa 7-1 bis etwa 20-1 beträgt, das Peptid das LHRH-Analogon ist, das p-Glu-His-Trp-Ser-Tyr-Ser-Tyr-D-Trp-Leu-Arg-Pro-Gly-NH₂ ist, und bei dem das Verhältnis von
(a) Einheiten auf l-Lactidbasis zu Einheiten auf Glykolidbasis etwa 75-25 bis etwa 90-10 beträgt, ,
(b) Einheiten auf d,l-Lactidbasis zu Einheiten auf Glykolidbasis etwa 75-25 bis etwa 90-10 beträgt, und
(c) Einheiten auf l-Lactidbasis zu Einheiten auf d,l-Lactidbasis etwa 80-20 beträgt.

26. Ummanteltes Mikroteilchen nach Anspruch 24, wenn abhängig von Anspruch 21, bei dem das Verhältnis von Glykolateinheiten zu Tartratresten in dem absorbierbaren Polymerkern etwa 7-1 bis etwa 20-1 beträgt, das Peptid das LHRH-Analogon ist, das p-Glu-His-Trp-Ser-Tyr-D-Trp-Leu-Arg-Pro-Gly-NH₂ ist, und bei dem das Verhältnis von
(a) Einheiten auf l-Lactidbasis zu Einheiten auf Glykolidbasis etwa, 75-25 bis etwa 90-10 beträgt,
(b) Einheiten auf d,l-Lactidbasis zu Einheiten auf Glykolidbasis etwa 75-25 bis etwa 90-10 beträgt, und
(c) Einheiten auf l-Lactidbasis zu Einheiten auf d,l-Lactidbasis etwa 80-20 beträgt.

27. Ummanteltes Mikroteilchen nach Anspruch 24, wenn abhängig von Anspruch 18, bei dem das Verhältnis von Glykolateinheiten zu Citratresten in dem absorbierbaren Polymerkern etwa 7-1 bis etwa 20-1 beträgt, das Peptid das Somatostatin-Analogon ist, das H-β-D-Nal-Cys-Tyr-D-Trp-Lys-Val-Cys-Thr-NH₂ ist, wobei die beiden Cys-Reste durch eine Disulfidbrücke verbunden sind, N-Hydroxyethylpiperazinylacetyl-D-Phe-Cys-Tyr-D-Trp-Lys-Abu-Cys-Thr-NH₂, wobei die beiden Cys-Reste durch eine Disulfidbrücke verbunden sind, oder N-Hydroxyethylpiperazinyl-ethylsulfonyl-Phe-Cys-Tyr-D-Trp-Lys-Abu-Gys-Thr-NH₂ ist, wobei die beiden Cys-Reste durch eine Disulfidbrücke verbunden sind,
und bei dem das Verhältnis von
(a) Einheiten auf l-Lactidbasis zu Einheiten auf Glykolidbasis etwa 75-25 bis etwa 90-10 beträgt,
(b) Einheiten auf d,l-Lactidbasis zu Einheiten auf Glykolidbasis etwa 75-25 bis etwa 90-10 beträgt, und
(c) Einheiten auf l-Lactidbasis zu Einheiten auf d,l-Lactidbasis etwa 80-20 beträgt.

28. Ummanteltes Mikroteilchen nach Anspruch 24, wenn abhängig von Anspruch 21, bei dem das Verhältnis von Glykolateinheiten zu Tartratresten in dem absorbierbaren Polymerkern etwa 7-1 bis etwa 20-1 beträgt, das Peptid das Somatostatin-Analogon ist, das H-β-D-Nal-Cys-Tyr-D-Trp-Lys-Val-Cys-Thr-NH₂, wobei die beiden Cys-Reste durch eine Disulfidbrücke verbunden sind, N-Hydroxyethylpiperazinyl-acetyl-D-Phe-Cys-Tyr-D-Trp-Lys-Abu-Cys-Thr-NH₂, wobei die beiden Cys-Reste durch eine Disulfidbrücke verbunden sind, oder N-Hydroxyethylpiperazinyl-ethylsulfonyl-Phe-Cys-Tyr-D-Trp-Lys-Abu-Cys-Thr-NH₂ ist, wobei die beiden Cys-Reste durch eine Disulfidbrücke verbunden sind,
und bei dem das Verhältnis von
(a) Einheiten auf l-Lactidbasis zu Einheiten auf Glykolidbasis etwa 75-25 bis etwa 90-10 beträgt,
(b) Einheiten auf d,l-Lactidbasis zu Einheiten auf Glykolidbasis etwa 75-25 bis etwa 90-10 beträgt, und
(c) Einheiten auf l-Lactidbasis zu Einheiten auf d,l-Lactidbasis etwa 80-20 beträgt.

29. Verfahren zur Herstellung eines ummantelten Mikroteilchens gemäß Anspruch 7, das die Stufe des Ummantelns eines gebundenen Mikroteilchens mit einem absorbierbaren Ummantelungspolymer umfasst.

30. Verfahren nach Anspruch 29, bei dem eine Dispersion der gebundenen Mikroteilchen in einer Lösung, die das absorbierbare Ummantelungspolymer und ein Lösungsmittel umfasst, auf ein vorgekühltes Medium getropft werden, wobei das Medium kein Lösungsmittel des absorbierbaren Ummantelungspolymers ist.

31. Verfahren nach Anspruch 30, bei dem die Lösung des absorbierbaren Ummantelungspolymers aus etwa 5 % bis 30 % des absorbierbaren Ummantelungspolymers besteht, das vorgekühlte Medium ein Alkohol mit zwei oder mehr Kohlenstoffatomen ist und die Temperatur des Mediums Raumtemperatur bis etwa -80°C ist.

32. Verfahren nach Anspruch 31, bei dem die Temperatur des vorgekühlten Mediums etwa -60°C bis -80°C beträgt und das Medium Isopropylalkohol ist.

33. Verfahren zur Herstellung eines ummantelten Mikroteilchens gemäß Anspruch 7, das die Stufe des Ummantelns eines gebundenen Mikroteilchens mit einem absorbierbaren Ummantelungspolymer unter Verwendung einer Emulsionstechnik umfasst.

## Revendications

1. Microparticule liée comprenant un coeur de polymère à hétérochaîne absorbable et un ou plusieurs peptides, une ou plusieurs protéines ou une combinaison de ceux-ci, immobilisés ioniquement sur ledit coeur de polymère à hétérochaîne absorbable,
- chaque peptide étant indépendamment choisi dans le groupe constitué par le peptide de libération de l'hormone de croissance (GHRP), l'hormone de libération de la lutéinostimuline (LHRH), la somatostatine, la bombésine, le peptide de libération de la gastrine (GRP), la calcitonine, la bradykinine, la galanine, l'hormone mélanotrope (MSH), le facteur de libération de l'hormone de croissance (GRF), l'amyline, les tachykinines, la sécrétine, l'hormone parathyroïdienne (PTH), l'enképhaline, l'endothéline, le peptide de libération du gène de la calcitonine (CGRP), les neuromédines, la protéine apparentée à l'hormone parathyroïdienne (PTHrP), le glucagon, la neurotensine, la corticotrophine (ACTH), le peptide YY (PYY), le peptide de libération du glucagon (GLP), le peptide intestinal vasoactif (VIP), le peptide d'activation de l'adénylate cyclase hypophysaire (PACAP), la motiline, la substance P, le neuropeptide Y (NPY), la TSH (thyrotropine) et les analogues et fragments de ceux-ci, ou un sel pharmaceutiquement acceptable dudit peptide ; et
- chaque protéine étant indépendamment choisie dans le groupe constitué par l'hormone de croissance, l'érythropoïétine, le facteur stimulant la formation de colonies de granulocytes, le facteur stimulant la formation de colonies de granulocytes ou de macrophages et les interférons.

2. Microparticule liée selon la revendication 1, dans laquelle ledit peptide, ladite protéine ou une combinaison de ceux-ci, ou un sel pharmaceutiquement acceptable de ceux-ci, représente 0,1% à 30% de la masse totale de la microparticule liée.

3. Microparticule liée selon la revendication 2, dans laquelle ledit coeur de polymère à hétérochaîne absorbable comprend des unités glycolate.

4. Microparticule liée selon la revendication 3, dans laquelle le coeur de polymère à hétérochaîne absorbable comprend en outre des restes citrate, des restes tartrate ou des restes malate.

5. Microparticule liée selon la revendication 4, dans laquelle le rapport d'unités glycolate audit reste citrate, audit reste tartrate ou audit reste malate est d'environ 7-1 à environ 20-1.

6. Microparticule liée selon la revendication 3, dans laquelle lesdites unités glycolate se terminent par une fraction carboxyle, ou une fraction amine.

7. Microparticule enrobée comprenant une ou plusieurs d'une microparticule liée enrobée à l'intérieur d'un polymère d'enrobage absorbable,
- ladite microparticule liée comprenant un coeur de polymère à hétérochaîne absorbable et un ou plusieurs peptides, une ou plusieurs protéines ou une combinaison de ceux-ci, immobilisés ioniquement sur ledit coeur de polymère à hétérochaîne absorbable ;
- chaque peptide étant indépendamment choisi dans le groupe constitué par le peptide de libération de l'hormone de croissance (GHRP), l'hormone de libération de la lutéinostimuline (LHRH), la somatostatine, la bombésine, le peptide de libération de la gastrine (GRP), la calcitonine, la bradykinine, la galanine, l'hormone mélanotrope (MSH) , le facteur de libération de l'hormone de croissance (GRF), l'amyline, les tachykinines, la sécrétine, l'hormone parathyroïdienne (PTH), l'enképhaline, l'endothéline, le peptide de libération du gène de la calcitonine (CGRP), les neuromédines, la protéine apparentée à l'hormone parathyroidienne (PTHrP), le glucagon, la neurotensine, la corticotrophine (ACTH), le peptide YY (PYY), le peptide de libération du glucagon (GLP), le peptide intestinal vasoactif (VIP), le peptide d'activation de l'adénylate cyclase hypophysaire (PACAP), la motiline, la substance P, le neuropeptide Y (NPY), la TSH (thyrotropine) et les analogues et fragments de ceux-ci, ou un sel pharmaceutiquement acceptable dudit peptide ; et
- chaque protéine étant indépendamment choisie dans le groupe constitué par l'hormone de croissance, l'érythropoïétine, le facteur stimulant la formation de colonies de granulocytes, le facteur stimulant la formation de colonies de granulocytes ou de macrophages et les interférons ; et
- ledit coeur de polymère à hétérochaîne absorbable comprenant des unités glycolate.

8. Microparticule enrobée selon la revendication 7, dans laquelle ledit peptide, ladite protéine ou ladite combinaison de ceux-ci ou ledit sel pharmaceutiquement acceptable de ceux-ci représente 0,1% à 30% de la masse totale de la microparticule liée, et où ledit coeur de polymère à hétérochaîne absorbable comprend en outre des restes citrate, des restes tartrate ou des restes malate.

9. Microparticule enrobée selon la revendication 8, dans laquelle le rapport des unités glycolate aux restes citrate, aux restes tartrate ou aux restes malate est d'environ 7-1 à environ 20-1 et lesdites unités glycolate se terminent par une fraction carboxyle ou une fraction amine.

10. Microparticule enrobée selon la revendication 9, dans laquelle ledit polymère d'enrobage absorbable comprend :
(a) des unités à base de 1-lactide et des unités à base de glycolide ;
(b) des unités à base de d,l-lactide et des unités à base de glycolide ;
(c) des unités à base de d,l-lactide ou
(d) des unités à base de l-lactide et des unités à base de d,l-lactide.

11. Microparticule enrobée selon la revendication 10, dans laquelle :
- le rapport des unités à base de 1-lactide aux unités à base de glycolide est d'environ 75-25 à environ 90-10 ; ou
- le rapport des unités à base de l-lactide aux unités à base de d,l-lactide est d'environ 80-20 ; ou
- le rapport des unités à base de d,l-lactide aux unités à base de glycolide est d'environ 75-25 à environ 90-10.

12. Microparticule enrobée selon la revendication 9, dans laquelle le polymère d'enrobage absorbable constitue 5 à 70% de la masse totale de la microparticule enrobée.

13. Microparticule enrobée selon la revendication 12, dans laquelle le polymère d'enrobage absorbable constitue 20-60% et de préférence 20-50%, de la masse totale de la microparticule enrobée.

14. Composition pharmaceutique comprenant les microparticules liées telles que définies à la revendication 1 et un support pharmaceutiquement acceptable.

15. Composition pharmaceutique comprenant les microparticules liées telles que définies à la revendication 1, un polyester liquide, formant un gel, absorbable, non aqueux, et facultativement un support pharmaceutiquement acceptable.

16. Composition pharmaceutique comprenant les microparticules enrobées telles que définies à la revendication 7 et un support pharmaceutiquement acceptable.

17. Composition pharmaceutique comprenant les microparticules enrobées telles que définies à la revendication 7, un polyester liquide, formant un gel, absorbable, non aqueux, et facultativement un support pharmaceutiquement acceptable.

18. Microparticule liée selon la revendication 3, dans laquelle ledit coeur de polymère à hétérochaîne absorbable comprend en outre des restes citrate et ledit peptide est un analogue de la LHRH ou un analogue de la somatostatine.

19. Microparticule liée selon la revendication 18, dans laquelle le rapport des unités glycolate aux restes citrate du coeur de polymère à hétérochaîne absorbable est d'environ 7-1 à environ 20-1, et où le peptide est ledit analogue de la LHRH qui est p-Glu-His-Trp-Ser-Tyr-D-Trp-Leu-Arg-Pro-Gly-NH₂.

20. Microparticule liée selon la revendication 18, dans laquelle le rapport des unités glycolate aux restes citrate est d'environ 7-1 à environ 20-1, et le peptide est ledit analogue de la somatostatine qui est H-β-D-Nal-Cys-Tyr-D-Trp-Lys-Val-Cys-Thr-NH₂, où les deux restes Cys sont liés par une liaison disulfure, N-hydroxyéthylpipérazinyl-acétyl-D-Phe-Cys-Tyr-D-Trp-Lys-Abu-Cys-Thr-NH₂, où les deux restes Cys sont liés par une liaison disulfure, ou N-hydroxyéthylpipérazinyl-éthylsulfonyl-Phe-Cys-Tyr-D-Trp-Lys-Abu-Cys-Thr-NH₂, où les deux restes Cys sont liés par une liaison disulfure.

21. Microparticule liée selon la revendication 3, dans laquelle le coeur de polymère à hétérochaîne absorbable comprend en outre des restes de tartrate et le peptide est un analogue de la LHRH ou un analogue de la somatostatine.

22. Microparticule liée selon la revendication 21, dans laquelle le rapport des unités glycolate aux restes tartrate est d'environ 7-1 à environ 20-1 et le peptide est ledit analogue de la LHRH qui le p-Glu-His-Trp-Ser-Tyr-D-Trp-Leu-Arg-Pro-Gly-NH₂.

23. Microparticule liée selon la revendication 21, dans laquelle le rapport des unités glycolate aux restes tartrate est d'environ 7-1 à environ 20-1 et le peptide est ledit analogue de la somatostatine qui H-β-D-Nal-Cys-Tyr-D-Trp-Lys-Val-Cys-Thr-NH₂, où les deux résidus Cys sont liés par une liaison disulfure, N-hydroxyéthylpipérazinyl-acétyl-D-Phe-Cys-Tyr-D-Trp-Lys-Abu-Cys-Thr-NH₂, où les deux restes Cys sont liés par une liaison disulfure, ou N-hydroxyéthylpipérazinyl-éthylsulfonyl-Phe-Cys-Tyr-D-Trp-Lys-Abu-Cys-Thr-NH₂, où les deux restes Cys sont liés par une liaison disulfure.

24. Microparticule enrobée comprenant une ou plusieurs microparticules liées telles que définies à l'une des revendications 18 ou 21, enrobée à l'intérieur d'un polymère d'enrobage absorbable qui comprend :
(a) des unités à base de l-lactide et des unités à base de glycolide ;
(b) des unités à base de d,l-lactide et des unités à base de glycolide ;
(c) des unités à base de d,l-lactide ; ou
(d) des unités à base de l-lactide et des unités à base de d,l-lactide.

25. Microparticule enrobée selon la revendication 24, lorsqu'elle est dépendante de la revendication 18, dans laquelle le rapport des unités glycolate aux restes citrate du coeur de polymère absorbable est d'environ 7-1 à environ 20-1, le peptide est ledit analogue de la LHRH qui est le p-Glu-His-Trp-Ser-Tyr-D-Trp-Leu-Arg-Pro-Gly-NH₂ et où le rapport :
(a) des unités à base de l-lactide aux unités à base de glycolide est d'environ 75-25 à environ 90-10 ;
(b) des unités à base de d,l-lactide aux unités à base de glycolide est d'environ 75-25 à environ 90-10 ; et
(c) des unités à base de l-lactide aux unités à base de d,l-lactide est d'environ 80-20.

26. Microparticule enrobée selon la revendication 24, lorsqu'elle est dépendante de la revendication 21, dans laquelle le rapport des unités glycolate aux restes tartrate du coeur de polymère absorbable est d'environ 7-1 à environ 20-1, le peptide est ledit analogue de la LHRH qui est p-Glu-His-Trp-Ser-Tyr-D-Trp-Leu-Arg-Pro-Gly-NH₂ et où le rapport :
(a) des unités à base de l-lactide aux unités à base de glycolide est d'environ 75-25 à environ 90-10 ;
(b) des unités à base de d,l-lactide aux unités à base de glycolide est d'environ 75-25 à environ 90-10 ; et
(c) des unités à base de l-lactide aux unités à base de d,l-lactide est d'environ 80-20.

27. Microparticule enrobée selon la revendication 24, lorsqu'elle est dépendante de la revendication 18, dans laquelle le rapport des unités glycolate aux restes citrate du coeur de polymère absorbable est d'environ 7-1 à environ 20-1, le peptide est ledit analogue de la somatostatine qui est H-β-D-Nal-Cys-Tyr-D-Trp-Lys-Val-Cys-Thr-NH₂, où les deux restes Cys sont liés par une liaison disulfure, N-hydroxyéthylpipérazinyl-acétyl-D-Phe-Cys-Tyr-D-Trp-Lys-Abu-Cys-Thr-NH₂, où les deux restes Cys sont liés par une liaison disulfure ou N-hydroxyéthylpipérazinyl-éthylsulfonyl-Phe-Cys-Tyr-D-Trp-Lys-Abu-Cys-Thr-NH₂, où les deux résidus Cys sont liés par une liaison disulfure ;
et où le rapport :
(a) des unités à base de 1-lactide aux unités à base de glycolide est d'environ 75-25 à environ 90-10 ;
(b) des unités à base de d,l-lactide aux unités à base de glycolide est d'environ 75-25 à environ 90-10 ; et
(c) des unités à base de l-lactide aux unités à base de d,l-lactide est d'environ 80-20.

28. Microparticule enrobée selon la revendication 24, lorsqu'elle est dépendante de la revendication 21, dans laquelle le rapport des unités glycolate aux restes tartrate du coeur de polymère absorbable est d'environ 7-1 à environ 20-1, le peptide est ledit analogue de la somatostatine qui est H-β-D-Nal-Cys-Tyr-D-Trp-Lys-Val-Cys-Thr-NH₂, où les deux restes Cys sont liés par une liaison disulfure, N-hydroxyéthylpipérazinyl-acétyl-D-Phe-Cys-Tyr-D-Trp-Lys-Abu-Cys-Thr-NH₂, où les deux restes Cys sont liés par une liaison disulfure, ou N-hydroxyéthylpipérazinyl-éthylsulfonyl-Phe-Cys-Tyr-D-Trp-Lys-Abu-Cys-Thr-NH₂, où les deux restes Cys sont liés par une liaison disulfure ;
et où le rapport :
(a) des unités à base de l-lactide aux unités à base de glycolide est d'environ 75-25 à environ 90-10 ;
(b) des unités à base de d,l-lactide aux unités à base de glycolide est d'environ 75-25 à environ 90-10 ; et
(c) des unités à base de l-lactide aux unités à base de d,l-lactide est d'environ 80-20.

29. Procédé pour fabriquer une microparticule enrobée telle que définie à la revendication 7, comprenant l'étape d'enrobage d'une microparticule liée par un polymère d'enrobage absorbable.

30. Procédé selon la revendication 29, dans lequel on fait tomber goutte à goutte une dispersion desdites microparticules liées dans une solution comprenant ledit polymère enrobage absorbable et un solvant sur un milieu pré-refroidi, ledit milieu n'étant pas un solvant dudit polymère d'enrobage absorbable.

31. Procédé selon la revendication 30, dans lequel la solution du polymère d'enrobage absorbable consiste en environ 5% à 30% du polymère d'enrobage absorbable, le milieu pré-refroidi est un alcool ayant deux atomes de carbone ou davantage et la température du milieu est la température ambiante à environ -80°C.

32. Procédé selon la revendication 31, dans lequel la température du milieu pré-refroidi est d'environ -60°C à -80°C et le milieu est l'alcool isopropylique.

33. Procédé pour fabriquer une microparticule enrobée telle que défini à la revendication 7, comprenant l'étape d'enrobage d'une microparticule liée par un polymère d'enrobage absorbable à l'aide d'une technique d'émulsion.
